# FASCICULE DE BREVET EUROPEEN

(11) **EP 2 356 210 B1**
(45) Date de publication et mention de la délivrance du brevet: **18.04.2018**
(21) Numéro de dépôt: 09804201.3
(22) Date de dépôt: 09.12.2009
(51) Int. Cl.: C12M 1/00

(54) **SYSTEME AUTOMATISE DE LYSE DE MICROORGANISMES PRESENTS DANS UN ECHANTILLON, D'EXTRACTION ET DE PURIFICATION DES ACIDES NUCLEIQUES DESDITS MICROORGANISMES AUX FINS D'ANALYSE**
AUTOMATISIERTES SYSTEM ZUR LYSE VON MIKROORGANISMEN IN EINER PROBE SOWIE ZUR EXTRAKTION UND REINIGUNG DER NUKLEINSÄUREN AUS DEN BESAGTEN MIKROORGANISMEN ZUR ANALYSE
AUTOMATED SYSTEM FOR THE LYSIS OF MICROORGANISMS IN A SAMPLE, AND FOR THE EXTRACTION AND PURIFICATION OF THE NUCLEIC ACIDS OF SAID MICROORGANISMS FOR ANALYSIS

(30) Priorité: 10.12.2008 FR 0858420
(43) Date de publication de la demande: 17.08.2011
(73) Titulaire: Biomérieux, 69280 Marcy L'etoile (FR)
(72) Inventeur: BROYER, Patrick, F-38500 Saint-Cassien (FR); DUPONTFILLIARD, Agnès, F-38190 Les Adrets (FR); GALDIERO, Massimo, I-50020 San Casciano Val di Pesa - Firenze (IT); GUY, Michel, F-38000 Grenoble (FR); KREUWEL, Hermanus, Johannes, Maria, NL-5481LW Schijndel (NL); MAIONE, Emiliano, I-50012 Bagno a Ripoli (FIRENZE) (IT); VERWIMP, Emiel, Gerebern, Maria, B-2460 Kasterlee (BE)
(86) Numéro de dépôt international: PCT/FR2009/052458
(87) Numéro de publication internationale: WO 2010/067019

(56) Documents cités:
- EP-A- 0 303 791
- WO-A-2008/104916
- WO-A2-2004/018704
- FR-A- 2 781 500
- GB-A- 203 402
- US-A1- 2002 185 557
- US-A1- 2005 070 944
- US-A1- 2007 064 521
- US-B1- 6 398 402

## Description

Le domaine technique de la présente invention est celui de l'analyse biologique. Plus particulièrement, la présente invention concerne en premier lieu un dispositif de lyse des microorganismes présents dans un échantillon environnemental ou clinique, d'extraction et de purification des acides nucléiques desdits microorganismes. L'invention concerne en outre un système automatisé de lyse des microorganismes d'extraction et de purification des acides nucléiques desdits microorganismes, aux fins d'analyse.

On observe, depuis plusieurs années, une recrudescence des infections nosocomiales au sein des hôpitaux. Ces infections s'expliquent par la contamination des personnes hospitalisées, et donc par définition immunodéprimées, par des microorganismes pathogènes présents dans la sphère environnementale hospitalière et non détruits malgré le soin toujours important apporté à la désinfection des outils et des surfaces et au traitement de l'air. Eu égard à ces cas de plus en plus fréquents de contaminations microbiologiques environnementales, la mise au point de dispositifs et de méthodes permettant d'améliorer et de faciliter les contrôles environnementaux est devenu un enjeu majeur pour les professionnels de santé.

Au-delà du problème des infections nosocomiales, le contrôle des conditions environnementales est également devenu depuis plusieurs années, un souci récurrent dans le milieu industriel, en particulier les industries agro-alimentaires, les industries pharmaceutiques ou cosmétiques. Dans les industries agro-alimentaires, on sait les conséquences désastreuses sur la santé des consommateurs, que peut avoir la contamination des produits, voire de matières premières, par un microorganisme pathogène. En effet, les toxi-infections alimentaires, dues à des bactéries telles que celles du genre *Listeria* ou *Salmonella* sont aujourd'hui monnaie courante. Le contrôle de la qualité de l'air est également un processus-clé dans la démarche qualité des industries pharmaceutiques ou cosmétiques.

Par ailleurs, ces contrôles doivent répondre à un niveau d'exigence toujours plus important, de par une règlementation toujours plus stricte.

Parmi les outils à disposition des professionnels de santé ou des industriels pour la réalisation de contrôles environnementaux, les aérobiocollecteurs sont des solutions de choix pour la détection des microorganismes de l'air. Ces dispositifs sont placés aux endroits appropriés dans les lieux où l'on souhaite mesurer l'aérobio-contamination. Ils sont généralement constitués d'un collecteur d'air couplé à un milieu de culture. L'air collecté par le collecteur d'air entre en contact avec le milieu de culture; milieu de culture sur lequel viennent se déposer les microorganismes éventuellement contenus dans l'air collecté. Le milieu de culture est ensuite récupéré et placé à l'étuve pour favoriser la croissance des microorganismes. Il est ainsi possible de détecter et d'identifier lesdits microorganismes par des techniques traditionnelles de microbiologie.

Ces dispositifs présentent néanmoins un inconvénient majeur qui est lié à la technologie utilisée. Cet inconvénient est le temps nécessaire pour obtenir le résultat d'analyse. En effet, l'utilisation des techniques traditionnelles de microbiologie, en particulier de bactériologie, implique le respect de temps d'incubation nécessaires à la croissance cellulaire, voire des phases de re-ensemencement sur des milieux de culture spécifiques pour permettre l'identification. Il s'ensuit que le temps nécessaire pour obtenir un résultat est relativement long, voire trop long, quand on cherche à détecter et identifier un organisme pathogène, responsable d'une infection nosocomiale ou d'une toxi-infection alimentaire.

Un autre inconvénient de ce type de dispositif est que l'utilisation de milieux de culture, s'il permet de discriminer entre les genres et espèces bactériennes, ne permet généralement pas de discriminer les souches d'une même espèce bactérienne. Or, l'on sait que la pathogénicité d'un microorganisme peut varier de manière significative selon la souche considérée.

Par ailleurs, ce type de dispositif présente comme inconvénient de ne pas permettre la mise en évidence des microorganismes présents dans l'air, viables mais non cultivables.

Il existe par ailleurs des dispositifs destinés à la récupération des particules présentes dans l'air, en particulier des microorganismes. Le document GB-2 254 024 décrit ainsi un dispositif pour collecter les particules contenues dans l'air dont le principe est basé sur l'effet cyclone. Si un tel dispositif se montre approprié à la collecte des particules contenues dans l'air, y compris les microorganismes, il n'est en aucun cas étudié pour traiter l'échantillon ainsi obtenu, en particulier pour réaliser l'extraction du matériel génétique destiné à être utilisé pour l'analyse.

De manière plus générale, les techniques les plus pertinentes en termes d'identification de microorganismes et/ou de rapidité de rendu des résultats, que ce soit vis-à-vis d'échantillons cliniques ou environnementaux, sont sans nul doute les techniques de diagnostic moléculaire. Ces techniques basées sur l'analyse du matériel génétique des microorganismes, et en particulier de certaines séquences spécifiques d'intérêt, permettent d'obtenir une identification très pointue des microorganismes en un temps record, puisqu'elles permettent de s'affranchir des étapes de culture.

Néanmoins, l'utilisation de telles techniques présente un certain nombre de limites, parmi lesquelles la plus importante est la quantité potentiellement limitée de microorganismes présents dans l'air et donc récupérable pour réaliser l'analyse. En effet, on sait que les prélèvements environnementaux, mais aussi certains prélèvements cliniques sont relativement pauvres en microorganismes. Il s'ensuit que la quantité de matériel génétique obtenue à partir de cette matière première est faible. Les performances de la technique utilisée pour extraire les acides nucléiques, en termes de rendement, deviennent alors un paramètre crucial.

Par ailleurs, la plupart des techniques existantes de lyse de microorganismes, ne sont pas générique vis à vis de l'ensemble des microorganismes et/ou , nécessitent l'intervention de personnel qualifié pour prendre en charge les étapes manuelles.

Le document WO-A-2005/038025 décrit une méthode d'extraction d'acides nucléiques de microorganismes prélevés notamment dans l'air. Cette méthode consiste dans la mise en oeuvre de trois méthodes de lyse différentes, à savoir une lyse chimique, une lyse par choc thermique et une lyse mécanique. Si une telle méthode permet sans nul doute d'optimiser le rendement d'extraction des acides nucléiques et donc d'augmenter la quantité de matériel génétique disponible pour l'analyse, il n'en demeure pas moins que ce rendement reste dépendant de la quantité de microorganismes récupérés. Or, rien dans ce document n'est décrit pour optimiser la récupération desdits microorganismes.

Le document US-5,707,861 décrit un dispositif permettant de désintégrer des cellules vivantes du type microorganismes. Ce dispositif permet de lyser les cellules en utilisant à la fois des billes de verre mais également l'effet de vibration dû à l'espace existant entre les tubes contenant les microorganismes et les trous du support portant lesdits tubes. Ainsi, un tel dispositif permet d'optimiser la lyse de cellules et donc d'optimiser l'extraction du matériel génétique. Un tel dispositif et la méthode mise en oeuvre par ce dernier présentent les mêmes limites que celles exposées précédemment, à savoir qu'ils restent dépendants de la quantité de microorganismes récupérés. Par ailleurs, ils présentent comme inconvénient supplémentaire de devoir effectuer subséquemment une étape de concentration des acides nucléiques afin de les isoler des débris cellulaires. Ils nécessitent enfin de récupérer manuellement les acides nucléiques, à l'issue de l'étape de concentration.

Ces problèmes se posent également avec le dispositif décrit dans le document US-5,567,050.

Le document EP 0 303 791 décrit un système et une méthode permettant de lyser des cellules au moyen d'un dispositif constitué par un broyeur à billes. Le broyeur est essentiellement constitué d'un rotor 3 et d'un stator 4, entre lesquels se situe un espace annulaire dans lequel intervient la lyse des cellules. Toutefois, un tel système n'est pas propice à la récupération des microorganismes, en particulier provenant d'un prélèvement d'air.

Des systèmes plus intégrés ont également été décrits. Ainsi, le document WO-A-2004/018704 décrit un dispositif, et une méthode associée utilisant la technique d'amplification PCR (Polymerase Chain Reaction), pour collecter des microorganismes dans l'air et les identifier. Ce système est spécialement adapté à la lutte contre les tentatives d'attentats par contamination biologique dans les centres de tri postal. Ce système se compose d'un dispositif de collecte d'air placé le long du circuit de transport du courrier, un dispositif de filtration/séparation des particules par effet cyclone, un dispositif de concentration/récupération des particules dans un échantillon liquide, un dispositif de transfert d'une fraction de l'échantillon dans une cartouche d'analyse par PCR GeneXpert™ de la société Cepheid. La cartouche est ensuite transférée manuellement dans un automate d'analyse biologique indépendant à fin d'identification du ou des microorganismes collectés dans l'air.

Si ce système permet de résoudre un bon nombre de problèmes techniques liés aux dispositifs et procédés décrits précédemment, il présente nonobstant des inconvénients majeurs. Le premier de ces inconvénients est que le système de traitement de l'échantillon (collecte, séparation, concentration/récupération) préalablement au transfert dans la cartouche d'analyse est relativement complexe et coûteuse. Un autre inconvénient est qu'il n'existe pas de cartouche GeneXpert™ pouvant faire à la fois la lyse et la purification des acides nucléiques. Un deuxième inconvénient est que les microorganismes collectés sont récupérés dans un échantillon liquide dont seule une fraction est analysée. Ce qui signifie que le risque de ne pas récupérer la totalité des microorganismes et donc la totalité des acides nucléiques est très important, limitant fortement la pertinence de l'analyse. Par ailleurs, malgré sa complexité, ce système nécessite le transfert manuel de la cartouche dans l'automate d'analyse GeneXpert™.

Ainsi, un premier objectif de la présente invention est de fournir un dispositif, un système et une méthode de lyse universelle, à la fois efficaces pour des prélèvements environnementaux et cliniques, pour une grande diversité de microorganismes, qu'il s'agisse de bactéries, de virus ou encore de champignons, éventuellement à l'état végétatif ou sous forme de spores.

Un autre objectif de la présente invention est de fournir un dispositif apte à lyser efficacement lesdits microorganismes contenus dans un prélèvement environnemental tel que l'air, ou dans un prélèvement clinique, afin d'en extraire les acides nucléiques et récupérer lesdits acides nucléiques à fin d'analyse, de manière intégrée.

Un autre objectif de la présente invention est de fournir un dispositif apte à collecter l'intégralité des microorganismes contenus dans un prélèvement d'air.

Un autre objectif de la présente invention est de fournir un dispositif simple de conception et minimisant le nombre et la complexité des étapes manuelles.

Un autre objectif de la présente invention est de fournir un dispositif excessivement compact.

Un autre objectif de la présente invention est de fournir un dispositif clos dans lequel les différentes étapes énoncées ci-dessus se déroulent sans risque de contamination extérieure.

Un autre objectif de la présente invention est de fournir un dispositif dans lequel lesdites étapes se déroulent sans transfert de l'échantillon par l'opérateur, empêchant par là même la contamination de ce dernier.

Un autre objectif de la présente invention est de fournir un dispositif et un système limitant l'intervention humaine et améliorant la traçabilité du traitement de l'échantillon.

Enfin, un autre objectif de la présente invention est de fournir un dispositif et un système automatisé en mesure de fournir des acides nucléiques cibles dans un tampon apte à être directement utilisé dans des étapes de diagnostic moléculaire comprenant par exemple des étapes d'amplification et de détection et ce, sans étapes de prétraitement additionnelles telles qu'une centrifugation ou une filtration.

Ces objectifs parmi d'autres sont atteints par la présente invention qui concerne en premier lieu, un dispositif de collecte de microorganismes contenus dans l'air, ledit dispositif comportant :
- un module de collecte d'air, comprenant :
   i. un élément supérieur comportant un conduit d'admission de l'air permettant l'entrée d'un flux d'air à l'intérieur dudit module, ledit conduit disposant à sa base, de moyens de perturbation du flux d'air,
   ii. un élément inférieur comportant des moyens d'évacuation de l'air permettant la sortie du flux d'air créé
   lesdits éléments supérieur et inférieur pouvant être solidarisés l'un à l'autre de sorte que le flux d'air puisse être créé à l'intérieur dudit module de collecte d'air ;
- une cartouche, de forme sensiblement cylindrique, comportant une zone de rétention des microorganismes, ladite zone de rétention comportant des moyens de lyse de microorganismes, ladite cartouche étant positionnée à l'intérieur dudit module de collecte d'air.

De façon préférentielle, la zone de rétention des microorganismes de la cartouche du dispositif de collecte de microorganismes comporte, en outre, un matériau apte à retenir les microorganismes, à maintenir les moyens de lyse en place et à se dissoudre en présence d'un liquide.

Dans le dispositif selon l'invention, les moyens de perturbation du flux d'air comportent un cône en forme d'ogive positionné au centre de la lumière du conduit d'admission de l'air et au moins une ailette reliant ladite pièce en ogive à la face interne du conduit d'admission d'air.

Avantageusement, le module de collecte d'air est destiné à être connecté à un circuit de recyclage de l'air ou à un dispositif d'aspiration de l'air.

Un autre objet de la présente invention concerne un dispositif de lyse de microorganismes, ledit dispositif comportant :
- une cartouche, de forme sensiblement cylindrique, comportant une zone de rétention des microorganismes, ladite zone de rétention comportant des moyens de lyse de microorganismes et un matériau apte à retenir lesdits microorganismes, à maintenir les moyens de lyse en place et à se dissoudre en présence de liquide, la dite cartouche jouant le rôle de stator ;
- un rotor, apte à être placé dans la cartouche, ledit rotor comportant des moyens de mise en rotation.

Selon un mode de réalisation préférentiel, ce dispositif comporte en outre une coiffe destinée à maintenir le rotor solidaire de la cartouche.

Avantageusement, les moyens de mise en rotation sont constitués par des rainures ménagées dans la paroi d'un conduit borgne, lesdites rainures étant diamétralement opposées et destinés à recevoir l'extrémité d'un axe de transmission d'un moyen d'entraînement dudit rotor en rotation.

Selon une caractéristique remarquable de ce dispositif de lyse de microorganismes, le diamètre interne de la cartouche est supérieur au diamètre externe du rotor, de sorte que lorsque le rotor est emboîté dans la cartouche, la distance séparant la paroi interne de la cartouche de la paroi externe du rotor est suffisamment importante pour permettre aux moyens de lyse de venir se positionner dans cet espace interstitiel et suffisamment faible pour que les moyens de lyse soient au contact de l'une ou l'autre desdites parois.

Un autre objet de la présente invention concerne un système de lyse de microorganismes et de purification des acides nucléiques desdits microorganismes, comportant :
- un moyen de positionnement du dispositif de lyse de microorganismes selon l'invention; un dispositif de collecte selon l'invention;
- un moyen d'entraînement du rotor en rotation, lorsque le dispositif de lyse est placé sur le moyen de positionnement ;
- au moins un conteneur de réception des acides nucléiques ;
- au moins un moyen d'aspiration/refoulement de liquide ;
- une vanne multivoies, en communication fluidique avec le dispositif de lyse de microorganismes, conteneur de réception des acides nucléiques purifiés et le moyen d'aspiration/refoulement de liquide.

Préférentiellement, ce système de lyse selon la revendication précédente, comporte en outre des moyens d'aimantation. Il peut également comporter des moyens de chauffage.

Selon un mode de réalisation particulier de l'invention, la vanne multivoies comporte un moyen d'aspiration-refoulement.

Un autre objet de l'invention concerne un procédé de collecte des microorganismes contenus dans l'air, ledit procédé comprenant les étapes consistant à :
a) Placer une cartouche selon l'invention, à l'intérieur d'un module de collecte d'air, selon l'invention, de sorte d'obtenir le dispositif de collecte de microorganismes selon l'invention, la zone de rétention à l'intérieur de ladite cartouche étant en communication avec le conduit d'admission d'air du module de collecte d'air,
b) Provoquer l'entrée d'air à l'intérieur dudit module de collecte d'air par tout moyen approprié,
c) Collecter les microorganismes contenus dans l'air dans la zone de rétention de la cartouche.

Un autre objet de l'invention concerne un procédé de lyse de microorganismes contenus dans l'air, ledit procédé comportant les étapes consistant à :
a) Placer une cartouche selon l'invention, à l'intérieur d'un module de collecte d'air, selon l'invention, de sorte d'obtenir le dispositif de collecte de microorganismes selon l'invention, la zone de rétention à l'intérieur de ladite cartouche étant en communication avec le conduit d'admission d'air du module de collecte d'air,
b) Provoquer l'entrée d'air à l'intérieur dudit module de collecte d'air par tout moyen approprié,
c) Collecter les microorganismes contenus dans l'air, dans la zone de rétention de la cartouche,
d) Sortir la cartouche du module de collecte d'air,
e) Placer le rotor selon l'invention, dans la selon l'invention, de sorte d'obtenir le dispositif de lyse de microorganismes selon l'une des revendications,
f) Positionner le dispositif de lyse de microorganismes dans le système de lyse de microorganismes et de purification des acides nucléiques,
g) Introduire un liquide d'élution dans la cartouche, entraînant la mise en suspension des moyens de lyse situés dans la zone de rétention des microorganismes de la cartouche, et
h) Lyser mécaniquement les microorganismes, par mise en rotation du rotor à l'intérieur de la cartouche grâce au moyen d'entraînement en rotation dudit rotor, ledit rotor entraînant en rotation les moyens de lyse des microorganismes.

Par liquide d'élution, on entend tout liquide apte à permettre la lyse, voire l'extraction et la récupération des acides nucléiques dans des bonnes conditions. Un tel liquide est généralement un tampon. Dans le cas où la mise en oeuvre du procédé selon l'invention a pour but d'extraire des acides nucléiques et de les récupérer à des fins d'analyse, un tel tampon devra permettre la conservation desdits acides nucléiques. De tels liquides sont bien connus de l'homme du métier.

Un autre objet de l'invention concerne un procédé d'extraction des acides nucléiques de microorganismes contenus dans l'air, ledit procédé comportant les étapes consistant à :
a) Placer une selon l'invention, à l'intérieur d'un module de collecte selon l'invention, de sorte d'obtenir le dispositif de collecte de microorganismes selon l'invention, la zone de rétention à l'intérieur de ladite cartouche étant en communication avec le conduit d'admission d'air du module de collecte d'air,
b) Provoquer l'entrée d'air à l'intérieur dudit module de collecte d'air par tout moyen approprié,
c) Collecter les microorganismes contenus dans l'air, dans la zone de rétention de la cartouche,
d) Sortir la cartouche du module de collecte d'air,
e) Placer le rotor dans la cartouche,
f) Introduire un liquide d'élution dans la cartouche à l'aide d'un moyen d'aspiration - refoulement, entraînant la mise en suspension des moyens de lyse contenus dans la cartouche, et
g) Lyser mécaniquement les cellules du tissu cellulaire, par mise en rotation du rotor, à l'intérieur de la cartouche grâce au moyen d'entraînement en rotation dudit rotor, ledit rotor entraînant en rotation les moyens de lyse, et
h) Aspirer le liquide d'élution contenant les acides nucléiques desdits microorganismes libérés lors de la lyse, et
i) Dispenser le liquide d'élution dans un conteneur de réception et de purification des acides nucléiques.

De façon préférentielle, les procédés de lyse des microorganismes et d'extraction des acides nucléiques décrits ci-dessus comportent une étape supplémentaire d') consistant à faire croître les microorganismes concentrés dans la zone de rétention de la cartouche.

Avantageusement, la mise en croissance est obtenue par incubation de la cartouche dans une étuve pendant un temps allant de 2 à 24 heures.

Préférentiellement, les étapes f) à i) des procédés de lyse des microorganismes et d'extraction des acides nucléiques décrits ci-dessus, sont mises en oeuvre dans le système de lyse des microorganismes et de purification des acides nucléiques.

Un autre objet de l'invention concerne un procédé de lyse de microorganismes, ledit procédé comportant les étapes consistant à :
a) Obtenir une cartouche dans laquelle des microorganismes sont concentrés dans la zone de rétention de ladite cartouche,
b) Placer le rotor selon l'invention, dans la cartouche, selon l'invention,
c) Introduire un liquide d'intérêt dans la cartouche, entraînant la mise en suspension des moyens de lyse situés dans la zone de rétention des microorganismes de la cartouche, et
d) Lyser mécaniquement les microorganismes, par mise en rotation du rotor, à l'intérieur de la cartouche, ledit rotor entraînant en rotation les moyens de lyse des microorganismes.

Un autre objet de l'invention concerne un procédé d'extraction des acides nucléiques de microorganismes, ledit procédé comportant les étapes consistant à :
a) Obtenir une cartouche dans laquelle des microorganismes sont concentrés dans la zone de rétention,
b) Placer le rotor selon l'invention, dans la cartouche, selon l'invention,
c) Introduire un liquide d'élution dans la cartouche, entraînant la mise en suspension des moyens de lyse situés dans la zone de rétention des microorganismes de la cartouche, et
d) Lyser mécaniquement les microorganismes, par mise en rotation du rotor, à l'intérieur de la cartouche, ledit rotor entraînant en rotation les moyens de lyse des microorganismes, et
e) Aspirer le liquide d'élution contenant les acides nucléiques desdits microorganismes libérés lors de la lyse, et
f) Dispenser le liquide d'élution dans un conteneur de réception et de purification des acides nucléiques.

Un autre objet de la présente invention concerne un procédé de lyse de microorganismes contenus dans un échantillon liquide, ledit procédé comportant les étapes consistant à :
a) Introduire l'échantillon liquide contenant lesdits microorganismes dans une cartouche, au voisinage de la zone de rétention, de sorte que ledit échantillon liquide entraîne la mise en suspension des moyens de lyse situés dans ladite zone de rétention des microorganismes de la cartouche,
b) Placer le rotor selon l'invention, dans la cartouche, selon l'invention
c) Lyser mécaniquement les microorganismes, par mise en rotation du l'intérieur de la cartouche, cette mise en rotation entraînant la mise en suspension des moyens de lyse situés dans ladite zone de rétention des microorganismes de la cartouche, ledit rotor entraînant en rotation les moyens de lyse sur lesquels sont retenus les microorganismes.

Par échantillon liquide, on entend tout échantillon liquide susceptible de contenir des microorganismes. Il peut s'agir d'un échantillon liquide d'origine humaine ou animal. Cet échantillon peut être par exemple, de l'urine, du sang total, du plasma ou tout autre liquide corporel. L'échantillon liquide peut être d'origine alimentaire tel qu'une boisson. Il peut être également d'origine environnementale, tel que de l'eau. Par ailleurs, l'échantillon liquide peut être également un liquide dit de transfert, dans lequel des éventuels microorganismes contenus sur un dispositif de prélèvement de surface, du type écouvillon tel que ceux commercialisés par la société COPAN, sous la dénomination flockedSWABS, ont été resuspendus par agitation dudit écouvillon dans ledit liquide de transfert.

Un autre objet de l'invention concerne un procédé d'extraction des acides nucléiques de microorganismes contenus dans un échantillon liquide, ledit procédé comportant les étapes consistant à :
a) Introduire un échantillon liquide contenant lesdits microorganismes dans une cartouche au voisinage de la zone de rétention, de sorte que ledit échantillon liquide entraîne la mise en suspension des moyens de lyse situés dans ladite zone de rétention des microorganismes de la cartouche,
b) Placer le rotor selon l'invention, dans la cartouche, selon l'invention,
c) Lyser mécaniquement les microorganismes, par mise en rotation du rotor, à l'intérieur de la cartouche, cette mise en rotation entraînant la mise en suspension des moyens de lyse situés dans ladite zone de rétention des microorganismes de ladite cartouche, ledit rotor entraînant en rotation les moyens de lyse sur lesquels sont retenus les microorganismes
d) Aspirer l'échantillon liquide contenant les acides nucléiques desdits microorganismes libérés lors de la lyse et
e) Dispenser l'échantillon liquide dans un conteneur de réception des acides nucléiques.

Un autre objet de la présente invention concerne un procédé d'extraction des acides nucléiques d'un échantillon de tissu cellulaire, ledit procédé comportant les étapes consistant à :
a) Introduire l'échantillon de tissu cellulaire dans la cartouche en présence d'un liquide d'élution,
b) Placer le rotor selon l'invention, dans la cartouche, selon l'invention,
c) Lyser mécaniquement les cellules du tissu cellulaire, par mise en rotation du rotor, à l'intérieur de la cartouche, ledit rotor entraînant en rotation les moyens de lyse contenus dans la cartouche,
d) Aspirer le liquide d'élution contenant les acides nucléiques desdits cellules, libérés lors de la lyse et
e) Dispenser le liquide d'élution dans un conteneur de réception des acides nucléiques.

Par échantillon tissulaire, on entend tout échantillon de tissu d'origine humaine ou animale à partir duquel il est possible d'extraire des acides nucléiques. Un tel échantillon peut être obtenu par biopsie par exemple d'un organe, d'un muscle, de peau. Ces tissus étant sains ou pathologiques, notamment tumoraux.

Il est à noter que tous les procédés d'extractions d'acides nucléiques selon la demande, peuvent présenter selon une variante avantageuse, une étape supplémentaire de purification de ces acides nucléiques, en vue d'un traitement subséquent tel qu'une amplification. Cette étape de purification, permettant la séparation entre les acides nucléiques et les autres constituants cellulaires relargués dans l'étape de lyse. Cette étape permet généralement de concentrer les acides nucléiques, et peut être adaptée à la purification d'ADN ou d'ARN. Elle peut être réalisée à l'aide de particules magnétiques. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet: WO-A-97/45202 et WO-A-99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep® Silica, Promega: MagneSil™ Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind™).

Par ailleurs, dans les différents procédés décrits ci-dessus, toutes les étapes postérieures à la reconstitution du dispositif de lyse de microorganismes, à savoir une fois le rotor placé dans la cartouche, peuvent être avantageusement mis en oeuvre dans le système de lyse des microorganismes et de purification des acides nucléiques.

Un autre objet de la présente invention concerne l'utilisation du dispositif de lyse de microorganismes selon l'invention, pour lyser des cellules d'un échantillon de tissu cellulaire.

Un autre objet de la présente invention concerne l'utilisation du système de lyse de microorganismes et de purification des acides nucléiques desdits microorganismes selon l'invention, pour lyser des cellules d'un échantillon de tissu cellulaire et purifier les acides nucléiques desdites cellules à des fins d'analyse.

Les microorganismes sont pris dans le groupe comprenant les bactéries, les virus, les levures, les moisissures, les parasites.

Les échantillons à partir desquels sont isolés les microorganismes sont d'origine environnementale. Ainsi, il peut s'agir d'un échantillon d'air ou de liquide, tel que de l'eau ; des prélèvement de surface. Les échantillons peuvent être également d'origine clinique, à savoir tout échantillon d'origine humaine ou animale, apte à faire l'objet d'une analyse pour la rechercher et l'identification d'un microorganisme, éventuellement pathogène.

La présence des acides nucléiques cibles peut être mise en évidence par la visualisation de réactions d'hybridation. On entend par réaction d'hybridation toute réaction entre un acide nucléique de capture et un acide nucléique cible isolé ou généré par une étape de transcription, de transcription inverse ou d'amplification de type NASBA (pour Nucleic Acid Sequence Based Amplification en anglais) ou PCR (pour Polymerase Chain Reaction en anglais).

On entend par acide nucléique, les oligonucléotides, les acides désoxyribonucléiques et les acides ribonucléiques, ainsi que leurs dérivés. Le terme oligonucléotide désigne un enchaînement d'au moins deux nucléotides (désoxyribonucléotides ou ribonucléotides, ou les deux), naturels ou modifiés, susceptibles de s'hybrider, dans des conditions appropriées d'hybridation, avec un oligonucléotide au moins partiellement complémentaire. Par nucléotide modifié, on entend par exemple un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine.

Pour illustrer une liaison internucléotidique modifiée, on peut mentionner les liaisons phosphorothioate, N-alkylphosphoramidate, alkylphosphonate et alkylphosphodiester.

Les alpha-oligonucléotides tels que ceux décrits dans FR-A-2 607 507, les LNA tels que phosphorothioate-LNA and 2'-thio-LNA décrits dans Bioorganic & Medicinal Chemistry Letters, Volume 8, Issue 16,18 August 1998 ,pages 2219-2222, et les PNA qui font l'objet de l'article de M. Egholm et al., J. Am. Chem. Soc. (1992), 114,1895- 1897, sont des exemples d'oligonucléotides constitués de nucléotides dont le squelette est modifié.

La visualisation des réactions d'hybridation peut être effectuée par tout moyen de détection, tels que des moyens directs ou indirects.

Dans le cas de la détection directe, c'est-à-dire sans l'intermédiaire d'un marquage, on observe les réactions d'hybridation par résonance plasmon ou par voltamétrie cyclique sur une électrode portant un polymère conducteur.

Dans le cas de la détection indirecte, c'est-à-dire par l'intermédiaire d'un marquage, le marquage peut être effectué soit directement sur les acides nucléiques cibles, soit par l'intermédiaire d'un partenaire de liaison spécifique desdits acides nucléiques préalablement marqué.

On entend par partenaire de liaison spécifique des acides nucléiques cibles tout partenaire capable de se lier avec l'acide nucléique cible et on donnera à titre d'exemples les acides nucléiques, les oligonucléotides ou polynucléotides et les substrats enzymatiques.

Par marquage, on entend la fixation d'un marqueur capable de générer directement ou indirectement un signal détectable. Une liste non limitative de ces marqueurs consiste en : les enzymes qui produisent un signal détectable par exemple par électrochimie, colorimétrie, fluorescence, luminescence, enzymes comme la péroxydase de Raifort (HRP), la phosphatase alcaline (PAL), la α-galactosidase, la glucose-6-phosphate déshydrogénase ; les inhibiteurs d'enzymes ; les co-facteurs d'enzymes ; les particules telles que les particules en or, les latex magnétiques, les liposomes ; les chromophores comme les composés, luminescents, colorants, les molécules radioactives comme le ³²P, le ³⁵S ou le ¹²⁵I, les molécules fluorescentes telles que la fluorescéine, la rhodamine, les Alexa®, l'umbelliférone, le luminol ou les phycocyanines. Dans le cas de la fluorescence, il peut s'agir du produit fluorescent d'une réaction enzyme-substrat, d'une combinaison fluorophore-quencher, d'une extinction de fluorescence ou de tout autre système basé sur des propriétés de fluorescence.

Des systèmes indirects peuvent être aussi utilisés, comme par exemple par l'intermédiaire d'un autre couple ligand/antiligand. Les couples ligand/antiligand sont bien connus de l'homme du métier, et on peut citer par exemple les couples suivants : biotine/streptavidine, sucre/lectine, polynucléotide/complémentaire du polynucléotide. Dans ce cas, c'est le ligand qui porte l'agent de liaison. L'antiligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR-A-2 781 802 ou WO-A-95/08000 de la demanderesse ou à l'article J. Histochem. Cytochem. 45 : 481-491, 1997.

Le marquage préalable des acides nucléiques cibles peut être effectué par incorporation directe ou indirecte de marqueur par une polymérase, par une kinase, de façon aléatoire ou spécifique, aux extrémités ou par incorporation « à l'intérieur » de la molécule.

Le marquage des partenaires de liaison spécifiques des analytes cibles est largement connu de l'homme du métier et est décrit par exemple par Greg T. Hermanson dans Bioconjugate Techniques, 1996, Academic Press Inc, 525B Street, San Diego, CA92101 USA.

Selon le type de marquage du conjugué utilisé, comme par exemple en utilisant une enzyme, l'homme du métier ajoutera des réactifs permettant la visualisation du marquage. Cette étape correspond à la révélation. Elle est précédée de l'utilisation d'un tampon de lavage qui permet d'éliminer les fractions d'analytes ou d'éléments non engagés dans la réaction, ou faiblement ou non spécifiquement liés, afin de limiter le bruit de fond.

Les buts et avantages du dispositif selon la présente invention seront mieux compris à la lumière de l'exemple nullement limitatif qui suit, en référence au dessin, dans lequel :
La figure 1 représente une vue en coupe longitudinale de la cartouche utilisée pour collecter et lyser les microorganismes.
La figure 2 représente une vue en coupe longitudinale du dispositif de collecte de microorganismes contenus dans l'air, selon un mode particulier de réalisation.
La figure 3 représente une vue partielle d'agrandissement en coupe longitudinale du dispositif de collecte de microorganismes tel que représenté sur la figure 1.
La figure 4 représente une vue en perspective de l'intérieur de l'élément supérieur du dispositif de collecte de microorganismes.
La figure 5 représente une vue partielle en perspective de l'intérieur de l'élément supérieur du dispositif de collecte de microorganismes tel que représenté sur la figure 3.
La figure 6 représente une vue en coupe longitudinale du dispositif de lyse des microorganismes.
La figure 7 représente une vue en coupe longitudinale de l'ensemble constitué du dispositif de lyse des microorganismes et de la vanne multivoies.
La figure 8A représente une vue en coupe longitudinale partielle du système de lyse de microorganismes au niveau de la vanne multivoies, dans une première configuration de cette dernière.
La figure 8B représente une vue en coupe longitudinale partielle du système de lyse de microorganismes au niveau de la vanne multivoies, dans une seconde configuration de cette dernière.

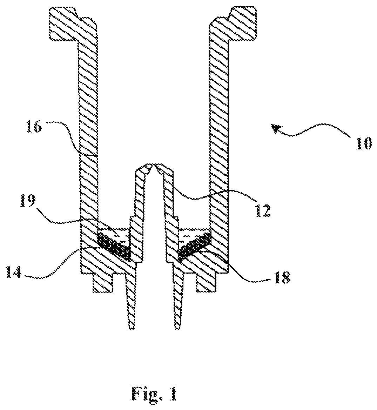

Une cartouche 10 représentée sur la figure 1 en coupe longitudinale, a la forme générale d'un cylindre de section transversale sensiblement circulaire. L'extrémité supérieure du cylindre est libre, alors que l'extrémité inférieure est constituée par une paroi, présentant en son centre un orifice, dans le prolongement duquel se trouve un conduit 12 s'étendant à l'intérieur de la cartouche 10. On constate que la section intérieure du conduit tend à se réduire au fur et à mesure que l'on se rapproche de son extrémité supérieure.

Comme on peut le voir sur la figure 1, la face interne de la paroi inférieure 14 de la cartouche 10 est inclinée, le point le plus bas de la pente se trouvant au contact du conduit 12 et le point le plus haut au contact de la paroi verticale 16 de la cartouche 10. Cette paroi 14 sert de support aux moyens de lyse 18 et constitue la zone de rétention des microorganismes. Ces moyens de lyse sont ici constitués par des billes de taille identique. Selon un mode de réalisation préférentielle, ces billes sont en verre. Elles pourraient néanmoins être constituées de tout autre matériau équivalent, tel que le fer. Ces billes présentent un diamètre avantageusement compris entre 200 et 800 micromètres (µm).

Selon une variante avantageuse, les billes peuvent être de tailles différentes. Il peut être ainsi particulièrement approprié d'utiliser un mélange de billes de diamètre compris entre 200 et 300 µm avec des billes dont le diamètre est compris entre 400 et 600 µm. De telles billes sont par exemple commercialisées par la société SIGMA sous la référence Acid-washed glass beads, 425-600µm, Ref : G8772.

Les billes sont maintenues en place sous la forme d'une ou plusieurs couches superposées au moyen d'une couche de matériau gélifié 19, disposée préférentiellement sur les billes. Le matériau gélifié doit répondre à plusieurs contraintes. La première est qu'il doit être inerte, afin de ne pas influer les procédés qui sont mis en oeuvre à l'intérieur de la cartouche. La deuxième est qu'il doit avoir la capacité de se dissoudre dans un liquide, pour la mise en oeuvre de la lyse des microorganismes. Un tel matériau peut par exemple un gel d'agarose (Sigma Aldrich, Agarose - Type I-B Low EEO, Réf.: A0576-25G). Le matériau gélifié peut également contenir du glycérol.

Alternativement, la matériau gélifié peut avantageusement être un milieu de culture pour les microorganismes. En effet, les milieux de culture gélosés sont classiquement utilisés dans le domaine du diagnostic in vitro et ce, depuis très longtemps. L'utilisation d'un tel milieu de culture présente plusieurs avantages. Le principal est de permettre une phase de croissance des microorganismes avant de réaliser la lyse de ces derniers. Même si le dispositif selon l'invention cherche à répondre à un besoin lié à la détection rapide de microorganismes pathogènes, il n'en demeure pas moins qu'une phase de croissance de quelques minutes à quelques heures permettrait une multiplication des microorganismes, ce qui a pour effet direct de disposer d'une plus grande quantité d'acides nucléiques (ceci permet de mettre à profit les délais inhérents à la récupération et au transfert des échantillons d'air collectés dans les salles devant être contrôlés jusqu'au démarrage de l'analyse proprement dite. Un second avantage de l'utilisation de milieux de culture est que ces derniers peuvent être sélectifs pour une ou plusieurs espèces données. Il s'ensuit que l'utilisation de tels milieux peut permettre une croissance et donc une détection sélective de certains microorganismes pathogènes au détriment d'autres microorganismes sans intérêt, qui peuvent éventuellement interférer dans l'analyse. Ainsi, il peut être envisagé de disposer de plusieurs cartouches différenciées, chacune étant adaptée à la détection d'une espèce spécifique de microorganisme.

Les dimensions de la cartouche 10 peuvent par exemple être de 8 à 16 mm pour ce qui est du diamètre interne, préférentiellement 12 mm. L'épaisseur totale des couches de billes est typiquement comprise entre 1 et 2 mm, ce qui correspond à une quantité de billes de verre comprise entre 0,1 et 1 gramme, préférentiellement 0,3 gramme.

La cartouche 10 est avantageusement réalisée en utilisant une technique de moulage par injection. Le matériau utilisé est par exemple du polypropylène, du polystyrène, du polycarbonate, des Cyclo-Olefines Cycliques (CoC) ou du PMMA ; le polycarbonate étant préféré.

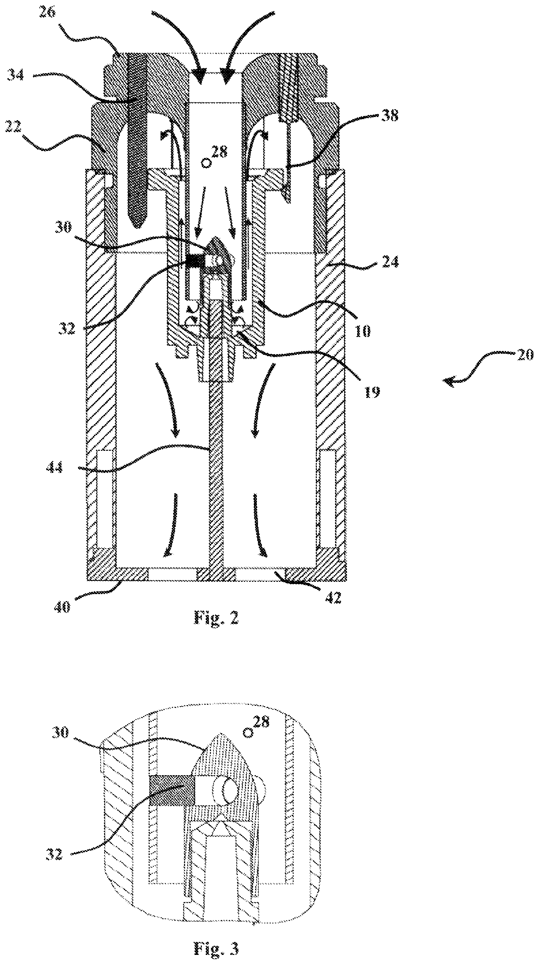

Le dispositif de collecte de microorganismes 20 est représenté sur la figure 2. Ce dispositif est constitué d'un module de collecte d'air à l'intérieur duquel est placée la cartouche 10. Ce module de collecte d'air est composé d'un élément supérieur 22 et d'un élément inférieur 24.

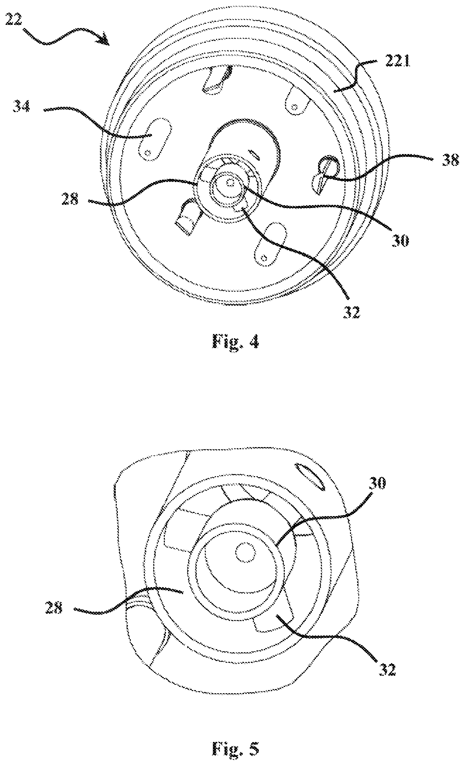

L'élément supérieur 22 est de forme générale cylindrique. Il est également représenté sur les figures 4 et 5, en perspective. L'extrémité inférieure de ce cylindre est libre, alors que l'extrémité supérieure est partiellement obturée par une paroi horizontale 26. Cette paroi 26 présente en son centre un orifice se prolongeant à l'intérieur de l'élément supérieur 22, par un conduit 28. Cette partie constitue en fait, le conduit d'admission de l'air à l'intérieur du module de collecte d'air 20. Selon un mode de réalisation particulier, ce conduit d'admission d'air peut être connecté à une canalisation d'un circuit de recyclage d'air par tout moyen approprié. A la base de ce conduit et au centre de ce dernier se trouve positionnée un cône 30 en forme d'ogive. Ce cône 30 est solidaire de la paroi du conduit 28 par le biais d'ailettes 32. Ces ailettes 32 sont bien visibles sur les figures 4 et 5. Sur le mode de réalisation tel que présenté sur ces figures, elles sont au nombre de trois. Leur nombre peut toutefois varié. Tel que représenté sur la figure 2, le rôle joué par l'ensemble cône 30 - ailettes 32 est de créer à la base du conduit 28, une perturbation du flux d'air qui pénètre dans le dispositif de collecte de microorganismes, de sorte que ce flux perturbé entre en contact avec le matériau gélifié de la zone de rétention des microorganismes, entraînant un creusement de ce dernier. Ce creusement permet d'améliorer de manière significative la capture des microorganismes contenus dans l'air.

Tel que représenté sur les figures 2 et 4, l'élément supérieur 22 comporte également trois pions centreurs 34 et trois pions de verrouillage 38. Les pions centreurs 34 sont destinés à assurer un centrage correct de la cartouche 10 par rapport à l'élément supérieur 22. Les pions de verrouillage 38 assurent un verrouillage de la cartouche en position centrée, sur l'élément supérieur 22. Ceci est bien représenté sur la figure 2.

L'élément supérieur 22 comporte dans sa partie basse un épaulement et une rainure réalisés sur tout le pourtour de la paroi verticale et destinés à faciliter la solidarisation de l'élément supérieur 22 et de l'élément inférieur 24.

L'élément inférieur 24 est également de forme générale cylindrique. L'extrémité supérieur de ce cylindre est libre, alors que l'extrémité inférieure est partiellement obturée, par une paroi horizontale 40 comportant en sa partie centrale des ouvertures d'évacuation 42 de l'air, sensiblement cylindrique. L'élément inférieur 24 comporte en outre un pion 44 de support et de positionnement de la cartouche 10. L'élément inférieur 24 vient alors se positionner sur l'élément supérieur 22. La cartouche 10 se retrouve positionner en appui sur l'élément inférieur 24, par l'intermédiaire du pion 44. En effet, lorsque la cartouche 10 est placée dans l'élément bas 24, l'élément 44 entre à l'intérieur du conduit 12 de la cartouche, plaçant et verrouillant ainsi cette dernière dans la bonne position.

L'extrémité supérieure de la paroi circulaire verticale de l'élément inférieur 24 peut comporter, sur sa face intérieure, une lèvre réalisée sur tout le pourtour de cette paroi. Cette lèvre est amenée à coopérer avec l'épaulement et la rainure ménagés dans l'élément supérieur 22 de façon à assurer le verrouillage entre l'élément supérieur 22 et de l'élément inférieur 24. L'emboîtement entre les éléments supérieur 22 et inférieur 24 est permis par une forme complémentaire de leur extrémité respectivement inférieure et supérieure. Il importe que cette solidarisation soit réversible.

Un moyen de solidarisation alternatif des éléments 22 et 24 peut être une solidarisation par vissage d'un élément sur l'autre. A cette fin, l'extrémité de la paroi d'un des éléments 22 ou 24 peut porter un filetage et l'extrémité de la paroi du deuxième élément un taraudage correspondant.

L'important est que le moyen de collecte soit clos de façon hermétique, afin d'éviter toute entrée parasite d'air.

Selon un mode particulier d'utilisation du moyen de collecte de l'air, l'extrémité inférieure du conduit d'évacuation de l'air 22 peut être connectée à une pompe d'aspiration de l'air (non représentée) ou tout moyen de pompage équivalent. Cette pompe permet d'aspirer l'air ambiant à l'intérieur du moyen de collecte de l'air, lorsqu'on souhaite par exemple réaliser une analyse de l'air ambiant dans un environnement déterminé, tel qu'une chambre d'hôpital, une pièce de production de produits pharmaceutiques ou de produits agroalimentaires. A cette fin, il peut être préférable de disposer d'un moyen de pompage fonctionnant de manière autonome et continu.

Le module de collecte d'air, 22 et 24, peut par exemple avoir un diamètre extérieur compris entre 10 et 40 mm, préférentiellement 20 mm. Le diamètre interne du conduit d'admission de l'air est par exemple de 6 mm.

Ce module de collecte d'air 10 peut avantageusement être réalisé dans un matériau apte à être stérilisé, en particulier par autoclavage. Il peut ainsi être en métal, tel que l'aluminium ou l'acier. Il peut également être en polymère tel que le polycarbonate, les Cyclo-Oléfines Cycliques (CoC) ou le PMMA.

Lorsque la circulation d'air dans le dispositif de collecte de microorganismes est déclenchée, le chemin suivi par ce dernier est représenté par les flèches sur la figure 2. Ainsi, on constate que l'air entre dans le dispositif de collecte de microorganismes par le conduit d'admission de l'air 28. Le conduit 12 de la cartouche 10 est partiellement inséré à l'intérieur du conduit d'admission de l'air 28, plus particulièrement dans le cône 30. Le flux d'air à la base du conduit 28 se transforme, au sommet du cône 30, en un flux périphérique. Par ailleurs, la présence du cône 30 empêche également l'air de pénétrer dans le conduit 12. Comme décrit *supra*, un flux d'air perturbé arrive sur le matériau gélifié 19 (billes 18 non représentées), entraînant à cet endroit la rétention par impaction des microorganismes transportés dans le flux d'air. Un tel processus est donc ainsi relativement similaire à celui qui se déroule dans les aérobiocollecteurs.

L'air, quant à lui, poursuit sa route aspiré par le moyen de pompage. Il remonte le long de la face interne de la paroi verticale 16 de la cartouche 10. Il redescend le long de la face externe de cette même paroi et s'échappe du module de collecte d'air 20 par les ouvertures d'évacuation 42.

Le débit d'air collecté dans le module de collecte d'air 10 peut être par exemple compris entre 20 et 100 litres/minute (l/min). Avantageusement, il est de 50 l/min.

Une fois l'étape de rétention/concentration des microorganismes à l'intérieur de la cartouche réalisée, cette dernière est extraite du module de collecte d'air par désolidarisation des éléments supérieur 22 et inférieur 24 dudit moyen, soit par déboîtement, soit par dévissage.

A ce stade, s'offre l'opportunité d'incuber la cartouche dans une étuve à 37°C, si l'on souhaite procéder à la mise en culture des microorganismes concentrés dans ladite cartouche. Comme déjà explicité *supra*, cette incubation dure généralement de quelques dizaines de minutes à quelques heures, pour ne pas allonger de manière trop importante la durée de l'analyse. Néanmoins, si l'obtention d'un résultat d'analyse n'a rien d'urgent, il peut être envisagé d'incuber la cartouche pendant une durée plus longue et plus en adéquation avec les contraintes de la croissance bactérienne, à savoir environ vingt-quatre heures.

De façon similaire, il peut également être envisagé de stocker la cartouche dans un environnement adapté à ce stockage, tel qu'une chambre froide, si l'on souhaite reporter l'analyse.

Selon une variante de l'invention, la cartouche 10 peut être fournie directement placée dans le module de collecte de l'air. Ce type de présentation a pour avantage d'éviter à l'utilisateur de devoir placer la cartouche dans le module de collecte d'air et donc ainsi réduire les risques de contamination lors de cette étape. Il est particulièrement avantageux dans ce cas de figure, que le module de collecte d'air soit produit dans le même matériau que la cartouche, à savoir en polypropylène, polycarbonate ou PMMA. Une fabrication par moulage par injection est alors particulièrement adaptée.

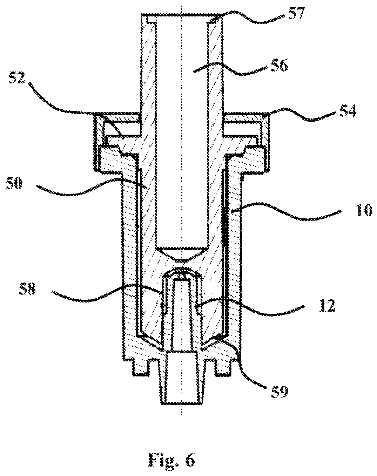

Lorsque la collecte des microorganismes, voire l'incubation, est réalisée, la cartouche 10 est solidarisée à un rotor 50 afin de constituer le dispositif de lyse de microorganismes, tel que représenté sur la figure 6. A cette fin, le rotor 50 est inséré dans la cartouche 10 par l'extrémité libre de cette dernière, selon un mouvement translatif vertical.

Le rotor 50, représenté en coupe longitudinale sur la figure 6, comporte un corps de forme générale cylindrique à section circulaire. Le corps présente sur la partie supérieure de sa paroi extérieure, une collerette 52 qui a pour fonction d'assurer l'étanchéité liquide de l'ensemble cartouche 10 - rotor 50, lorsque ce dernier est complètement inséré dans la cartouche 10, en venant en appui sur l'extrémité supérieure de la paroi verticale de la cartouche 10.

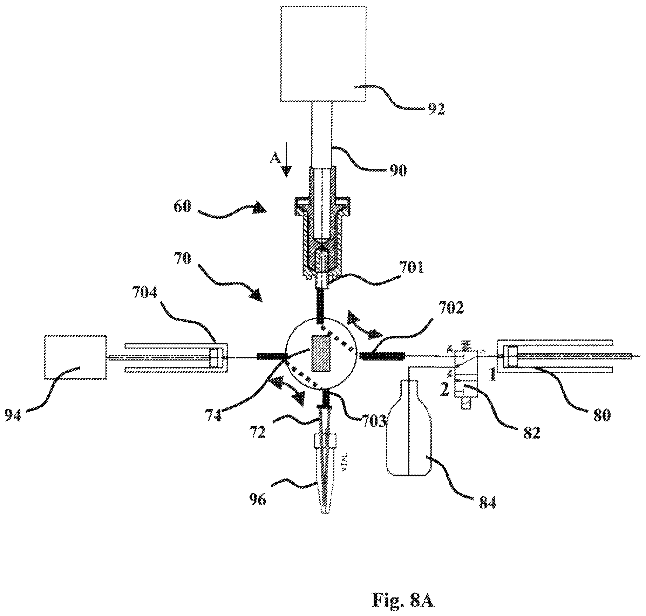

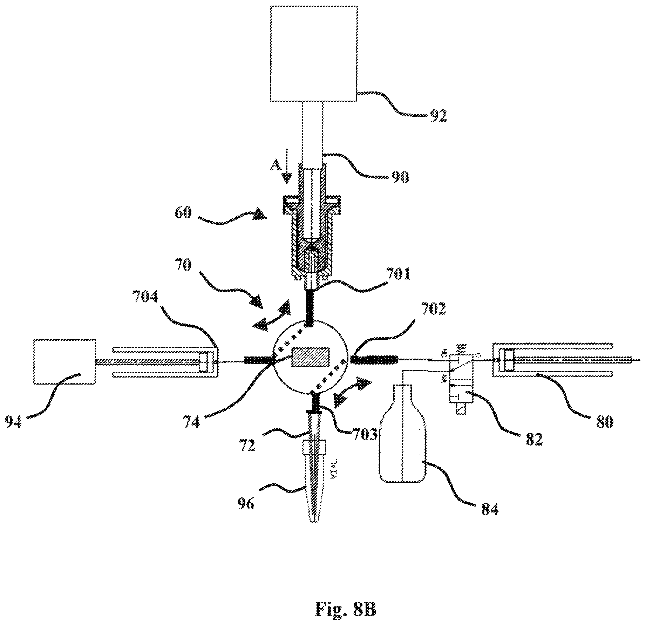

Afin de renforcer l'étanchéité, une coiffe 54 peut être fixée sur la cartouche 10 par tout moyen approprié de façon à maintenir le rotor 50 solidaire de la cartouche. Par exemple, la coiffe 54 peut être fixée par emboîtement ou par vissage. La coiffe est de forme sensiblement annulaire et vient s'enfiler sur la partie haute du rotor constituant le conduit borgne 56, ménagé dans le rotor. A l'extrémité supérieure de ce conduit borgne, sont réalisées dans la paroi deux rainures 57, diamétralement opposées. Ces rainures ont pour fonction de recevoir l'extrémité d'un axe de transmission 90 d'un moyen d'entraînement 92 dudit rotor en rotation, tels que représentés sur les figures 8A et 8B. L'extrémité de cet axe présente ainsi une forme complémentaire de ces rainures, sous forme d'une nervure qui vient s'insérer dans ces rainures. L'axe d'entraînement peut être celui d'un moteur électrique. Alternativement, il peut être une partie d'un dispositif de mise en rotation manuel.

Comme représenté sur la figure 6, le rotor 50 présente, dans sa partie inférieure, des formes extérieures parfaitement complémentaires des formes intérieures de la cartouche 10. Il s'ensuit que le rotor 50 vient parfaitement se positionner à l'intérieur de la cartouche 10, le conduit 12 jouant le rôle de guide en venant s'insérer dans une cavité 58, ménagée à la base du rotor 50. Cette cavité 58 est de forme sensiblement cylindrique à section circulaire et présente une extrémité supérieure de forme sensiblement conique. Le positionnement du rotor 50 se produit jusqu'à ce que sa paroi inférieure 59 se retrouve en appui sur l'ensemble constitué par les billes 18 et le matériau gélifié 19.

Le rotor 50 et la coiffe 54 sont avantageusement réalisés dans le même matériau polymère que la cartouche, à savoir en polypropylène, polycarbonate ou PMMA. De façon préférentielle, ces pièces sont réalisées moulées par injection dans du polycarbonate transparent (Makrolon® 2858, Bayer).

L'ensemble cartouche 10 - rotor 50 - coiffe 54 constitue donc le dispositif de lyse de microorganismes 60 selon l'invention et sera dénommé dispositif de lyse *infra.*

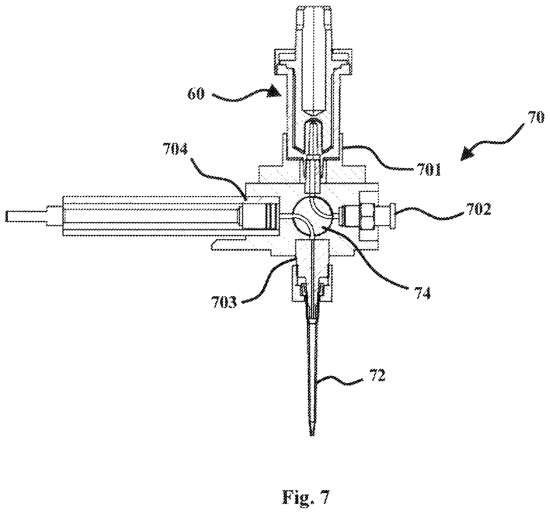

Une fois le dispositif de lyse constitué, il est placé sur une vanne 4-voies, tel que représenté sur la figure 7. Cette vanne 70 comporte une première voie 701 destinée à recevoir le dispositif de lyse 60. La connexion dudit dispositif sur la vanne se fait par la base du conduit 12 de la cartouche 10. La deuxième voie comporte un orifice de connexion 702, destiné à être connecté à un premier moyen d'aspiration-refoulement, partie du système de lyse des microorganismes et de purification des acides nucléiques, selon l'invention. Avantageusement, ce moyen d'aspiration-refoulement est une pompe, également connectée au récipient contenant la solution de tampon d'élution. Une telle pompe est représentée schématiquement sur les figures 8A et 8B. Son fonctionnement sera explicité *infra.* La troisième voie comporte un orifice de connexion 703 sur lequel est fixé un cône jetable 72. Enfin, la quatrième voie est constituée par un second moyen d'aspiration-refoulement 704. Ce second moyen est un seringue qui fait bloc avec la vanne. Cette seringue 704 est amenée à coopérer avec un moyen d'actionnement translatif faisant partie du système de lyse des microorganismes et de purification des acides nucléiques selon l'invention et destiné à mouvoir le piston de ladite seringue. Enfin, la vanne 70 comporte un axe central 74 comportant deux voies fluidiques. Tel que représenté sur la figure 7, ces deux voies assurent la connexion respectivement entre, d'une part le dispositif de lyse 60 et l'orifice de connexion 702 ; et d'autre part, entre la seringue 704 et l'orifice de connexion 703 sur lequel est fixé le cône jetable 72. L'axe 74 peut être mû en rotation d'un quart de tour, de sorte que les connexions fluidiques sont changées. L'orifice de connexion 702 se retrouve connecté à l'orifice de connexion 703 et le dispositif de lyse 60 se retrouve connectée à la seringue 704.

L'ensemble constitué par le dispositif de lyse 60 et la vanne 70 constitue la partie consommable. Il est ensuite placé sur le système de lyse des microorganismes et de purification des acides nucléiques. Il s'agit d'un automate comportant trois fonctions principales distinctes : une fonction lyse, une fonction de transfert de liquide et une fonction de purification. Ces fonctions sont décrites *infra.*

La mise en place de l'ensemble dispositif de lyse 60 - vanne 70 dans le système de lyse des microorganismes et de purification des acides nucléiques est réalisée de la façon suivante. En premier lieu, un emplacement est dédié à la mise en place de cet ensemble. Cet emplacement se caractérise par le fait qu'il comporte des moyens de fixation de la vanne 70. Ces moyens peuvent par exemple être constitués par des bras ou des mâchoires entre lesquels la vanne est insérée en force.

Une fois l'ensemble dispositif de lyse 60 - vanne 70 positionné, les différentes connexion avec la vanne sont réalisées. Ceci est représenté schématiquement sur les figure 8A et 8B. Ainsi, la première connexion se fait par l'orifice de connexion 702 de la vanne 70. Il s'agit d'une connexion *in fine* avec le moyen d'aspiration-refoulement 80 qui se trouve à demeure sur l'automate est connecté à l'orifice de connexion 702. Comme explicité *supra*, ce moyen d'aspiration-refoulement 80 se présente préférentiellement sous la forme d'une pompe fixe, part intégrante de l'automate non représenté en totalité sur les figures 8A et 8B. De manière plus précise, la pompe 80 est en fait connectée à une vanne 82 également placée à demeure sur l'automate. Cette vanne fixe 82 est par ailleurs en connexion fluidique, d'une part avec la vanne 70 par le biais de l'orifice de connexion 702, et d'autre part avec un réservoir 84 contenant un tampon dit d'élution. La vanne fixe 82 permet ainsi de connecter la pompe fixe 80, soit à la vanne 70, soit au réservoir 84, selon sa position.

L'ensemble dispositif de lyse - vanne est également relié à l'arbre de transmission 90 du moyen d'entraînement en rotation 92 du rotor du dispositif de lyse. Il est à noter qu'une fois cette connexion réalisée, l'arbre de transmission 90 exerce sur le rotor une pression selon une composante verticale conformément à la flèche A sur la figure 8A, de sorte que cette pression se transmet du rotor sur l'ensemble constitué par les billes 18 et le matériau gélifié 19.

La troisième connexion entre l'ensemble dispositif de lyse - vanne et l'automate consiste dans la connexion entre la seringue 704 de la vanne 70 et le moyen d'actionnement 94 de ladite seringue. Ce moyen d'actionnement est un moyen d'actionnement translatif, tel un ensemble constitué par un moteur pas à pas et une vis sans fin et permet soit de tirer, soit de pousser le piston de la seringue 704, et ainsi, soit d'aspirer un liquide à l'intérieur de la seringue, soit de refouler un liquide contenu dans la seringue.

Enfin, la quatrième connexion entre l'ensemble dispositif de lyse - vanne et l'automate concerne le moyen d'actionnement en rotation de l'axe 74 de la vanne 70. Ce moyen n'est pas représenté sur les figures 8A et 8B. Il s'agit d'un moteur apte à faire tourner un axe de transmission sur lui-même, cet axe de transmission étant connecté à l'axe 74 de la vanne 70.

Lors de la mise en oeuvre du protocole de lyse des microorganismes collectés dans la cartouche 10, la vanne 70 est configurée de telle manière que le dispositif de lyse 60 soit en connexion fluidique avec l'ensemble constitué par la pompe 80 et la vanne fixe 82. La seringue 704 est alors en connexion fluidique avec l'orifice de connexion 703 sur lequel est fixé le cône jetable. La vanne fixe 82 est initialement configurée de telle manière que la pompe 80 est en connexion fluidique avec le réservoir 84 du tampon d'élution. La pompe 80 aspire alors une quantité déterminée de tampon d'élution. La vanne fixe 82 est alors changée de configuration de sorte que la pompe 80 devient connectée fluidiquement au dispositif de lyse 60 par le biais de la vanne 70. La pompe 80 refoule alors le volume de tampon d'élution prélevé, qui chemine jusque dans le dispositif de lyse 60 et plus particulièrement dans l'espace interstitiel entre la paroi interne de la cartouche 10 et la paroi externe du rotor 50 (cf. figure 6). Le tampon d'élution mouille alors le matériau gélifié 19 et les billes 18. Le matériau gélifié 18 est alors dissous par l'action conjuguée du tampon d'élution et d'une mise en rotation lente du rotor 50. Les billes se trouvent alors en suspension dans ce dernier. Cette mise en suspension, couplé à l'effet de pression exercée par le rotor et la mise en rotation de ce dernier, entraîne le déplacement d'une fraction des billes 18 dans l'espace interstitiel entre la paroi interne verticale de la cartouche 10 et la paroi externe verticale du rotor 50. Il est à noter que cet espace interstitiel présente préférentiellement une largeur comprise 600 et 800 µm. Cette largeur est directement liée au diamètre des billes 18 utilisées. En effet, les billes doivent, à la fois, pouvoir circuler facilement dans cet espace et pouvoir être mues en rotation par la mise en rotation du rotor 50. A cette fin, la paroi verticale externe du rotor peut présenter une surface rugueuse, ce qui facilite la mise en rotation des billes.

Une fois, les billes réparties dans la zone de rétention de la cartouche et le long de la paroi verticale du rotor, ce dernier peut alors être complètement inséré dans la cartouche.

Bien entendu, les microorganismes retenus tant sur le matériau gélifié que sur les billes sont transférés dans l'espace interstitiel au même titre que les billes 18, par le flux du tampon d'élution.

Une fois, les billes 18 réparties le long de la paroi verticale externe du rotor 50, l'étape de lyse proprement dite est initiée. Le rotor 50 est alors mû en rotation grâce au moyen d'entraînement en rotation/moteur 92 et par l'intermédiaire de l'arbre de transmission 90. Il peut néanmoins être envisagé de mettre en rotation le rotor de façon manuelle, dans un protocole simplifié n'utilisant pas le système de lyse des microorganismes et de purification des acides nucléiques.

A titre d'exemple, les valeurs de vitesse de rotation peuvent être comprises entre 200 et 3000 tours/min. Préférentiellement, le rotor est initialement mis en rotation à une vitesse lente de 200 tours/min à fins de répartition et d'homogénéisation des billes et du tampon d'élution dans l'espace interstitiel. Cette mise en rotation lente dure environ 15 secondes.

Ensuite, la vitesse de rotation augmente pour atteindre une valeur comprise entre 1000 et 3000 tours/min durant un temps compris entre 1 et 5 minutes. C'est pendant cette période que s'effectue la lyse des microorganismes éventuellement collectés dans l'air.

Il est bien évident que le choix de la vitesse et du temps de rotation du rotor est fonction du type de microorganismes que l'on cherche à lyser.

Durant cette étape, les billes 18 sont mises en rotation autour de leur axe de symétrie par frottement contre la surface externe verticale du rotor et la surface interne verticale de la cartouche. Cette double rotation entraîne la lyse mécanique des microorganismes qui se trouvent emprisonnés entre les billes 18 et les surfaces respectives du rotor ou de la cartouche. Il en résulte une libération des acides nucléiques dans le tampon d'élution.

Une fois l'étape de lyse achevée et les acides nucléiques libérés, le tampon d'élution contenant les acides nucléiques doit être aspiré. La vanne 70 change alors de configuration par mise en rotation de son axe 74, de sorte que le dispositif de lyse 60 et la seringue 704 se retrouvent en communication fluidique, tel que représenté sur le figure 8B. Le piston de la seringue 704 est alors mû en translation grâce au moyen d'actionnement 94, afin d'aspirer le tampon d'élution contenant les acides nucléiques à l'intérieur de ladite seringue 704. Cette étape d'aspiration se déroule alors que le rotor 50 est toujours mû en rotation, mais à basse vitesse (environ 200 tours/min).

La vanne 70 repasse alors dans sa configuration initiale, tel que représenté sur la figure 8A, de sorte que la seringue 704 est en communication fluidique avec l'orifice de connexion 703. Le tampon d'élution contenant les acides nucléiques est alors prêt à être dispensé dans un tube par le biais du cône 72, à fins de purification desdits acides nucléiques. En effet, le lysat contenu dans le tampon d' élution contient effectivement les acides nucléiques, mais également tous les débris cellulaires.

Pour ce faire, un tube d'analyse 96 est amené à proximité du cône 72. Avantageusement, l'automate dispose d'un support pour recevoir un tel tube. En l'occurrence, un tel support peut être lié à un bras qui peut être mû entre un position basse ou position de repos, dans laquelle le tube peut être inséré dans le support et une position haute ou position de travail dans laquelle le tube est à proximité du cône 72 (tel que représenté sur les figures 8A et 8B). Bien entendu, il est préférable que le cône 72 pénètre dans le tube 96. Le tube 96 comporte un tampon dit de lyse et des particules de silice magnétique, nécessaire à la purification des acides nucléiques. De telles particules ont une diamètre compris entre 1 et 3 µm. Par ailleurs, un septum pré-percé (non représenté) est placé au niveau de l'orifice du tube 96 afin d'éviter toute contamination de ce dernier. Lors de la montée du tube 96, le cône 72 traverse le septum par le pré-perçage. Le tampon d'élution avec le lysat est alors dispensé dans le tube d'analyse. A ce stade, il est préférable d'utiliser la seringue 704 pour réaliser plusieurs aspirations/refoulements, et ce afin d'homogénéiser le lysat avec le tampon de lyse et les particules magnétiques. La fonction du tampon de lyse est de stabiliser les acides nucléiques (protection contre les nucléases) et d'ajuster le pH à une valeur optimale pour la phase de capture.

Si le mélange est correctement homogénéisé, le tube peut alors être mis au repos pour permettre la capture passive des acides nucléiques par les particules magnétiques. Le temps de mise au repos peut être par exemple de 2 minutes. Dans le cas où l'homogénéisation n'est pas correcte ou en absence d'homogénéisation, la capture des acides nucléiques va être réalisée de manière active. En effet, le tube et les particules magnétiques qu'il contient vont être soumis à des champs magnétiques d'orientations différentes. Pour ce faire, le tube est placé en position basse puis est mis au contact d'aimants par le biais du bras le portant. Ces aimants peuvent être positionnées sur deux plaques parallèles solidaires du bras portant le tube. Ces deux plaques sont mobiles en translation horizontale au moyen d'un moyen de déplacement *ad hoc* et sont ainsi déplacées de façon à ce que le tube se retrouve entre lesdites plaques. Le nombre d'aimants sur chaque plaque est variable. Il est avantageux que les aimants soient à des hauteurs différentes sur les plaques. De même, il est important que les aimants sur une plaque ne soient pas placés en face des aimants sur l'autre plaque, mais soient décalés relativement au sens de déplacement du tube selon une séquence déterminée. Ainsi, lors du déplacement des plaques, le tube est soumis au fur et à mesure, soit au champ magnétique d'un aimant sur la première plaque, soit au champ magnétique d'un aimant sur la deuxième plaque. Ces champs pouvant être positionnés à une hauteur différente relativement au chemin suivi par les plaques. Il s'ensuit que les particules magnétiques, se trouvant attirer par ces aimants, se mettent en amas, et que cet amas est amené à se déplacer dans le tube, en fonction de l'attraction exercée par les aimants et surtout de leur position par rapport au tube. Il est à noter que ce positionnement relatif des aimants par rapport au tube d'analyse peut être obtenu également par un déplacement du tube selon un axe vertical ; auquel cas, les aimants sont tous placés à la même hauteur et selon une séquence déterminée. Dans le cas d'une capture active des acides nucléiques, le temps dédié à ladite capture sera plus important (entre 4 et 6 minutes).

Une fois la capture des acides nucléiques effectuée, une ou plusieurs étapes successives de lavage sont mises en oeuvre. A cette fin, le tube est placé en regard d'un aimant en position haute de sorte d'accumuler les particules magnétiques portant les acides nucléiques dans la partie haute du tube contre la paroi verticale. Le tube est alors amené en position haute de sorte que la cône 72 entre en contact avec le liquide contenu dans le tube. L'aspiration du liquide contenu dans le tube se fait alors dans la seringue 704 par actionnement de son piston. La seringue 704 sert alors de récipient de stockage des déchets liquides aspirés dans le tube. Afin de procéder au lavage des acides nucléiques, la vanne 74 est mise dans la configuration dans laquelle l'orifice de connexion 702, et donc la pompe 80, est en communication fluidique avec l'orifice de connexion 703. La vanne 82 est mise en position permettant l'aspiration de tampon d'élution par la pompe 80. Le volume de tampon d'élution aspiré est par exemple de 300µl. La position de la vanne 82 est ensuite modifiée pour permettre à la pompe 80 de dispenser le tampon d'élution dans le tube 96. Afin d'assurer un lavage optimal, le tube est redescendu en position basse et un déplacement des plaques portant les aimants est à nouveau réalisé afin d'optimiser les échanges entre les particules et le tampon d'élution. Les plaques sont susceptibles d'effectuer plusieurs allers-retours et ce, à une vitesse de passage variable selon les besoins. L'aspiration du tampon d'élution contenu dans le tube est ensuite réalisée au moyen de la seringue 704, tel que décrit précédemment. De nouvelles étapes de lavage peuvent être éventuellement réalisées de manière identique. Il est à noter que le piston de la seringue 704 est actionné par pallier, de sorte que la seringue 704 se remplit au fur et à mesure avec le liquide aspiré dans le tube d'analyse, qu'il s'agisse du tampon de lyse ou de tampon d'élution utilisé pour les lavages. Une fois les acides nucléiques transférés dans le tube d'analyse, la seringue 704 est ensuite uniquement dévolue au rôle du stockage des déchets liquides, ce qui représente un intérêt considérable. Par ailleurs, il est à noter que la pompe 80 ne sert qu'à la dispense de tampon d'élution et n'est jamais au contact de matière « souillée ». Cet aspect est également particulièrement important sachant que la pompe 80 et la vanne 82 reste à demeure sur l'automate, contrairement à la seringue 704 qui est une partie intégrante de la vanne 74, qui a pour vocation d'être à usage unique.

Une fois, le lavage des acides nucléiques réalisés, la dernière aspiration du tampon d'élution dans le tube 72 n'est que partielle. En effet, un volume déterminé de tampon d'élution est conservé dans le tube et constitue le volume d'élution dans lequel les acides nucléiques vont être élués, c'est à dire dissociés des particules magnétiques. Le volume d'élution peut varier entre 5 et 150µl, il est préférentiellement de 25 ou de 40 µl. Pour cette ultime aspiration, un amas de particules magnétiques est formé dans la partie basse du tube, en mettant ce dernier en regard d'un aimant en position basse. L'aspiration se fait par contre à la surface du liquide et ce afin, d'éviter tout aspiration de particules.

Dans le volume restant, on procède alors à l'élution des acides nucléiques. Pour ce faire, le tube est chauffé à 75°C à l'aide d'un module de chauffage, intégré au support du tube, jusqu'à atteindre une température de 65°C dans le liquide durant 5min environ. Simultanément à l'étape de chauffage, le tube est soumis aux aimants situés en position basse, pour favoriser l'élution.

Une fois les acides nucléiques purifiés et élués dans le volume d'élution, ceux-ci peuvent être amplifiés à fins d'analyse. Pour cela, tout ou partie du volume d'élution doit être mis en contact avec le tampon dit de mise en condition de l'amplification (40mM Tris HCl, pH 8,5, 12mM MgCl2, 70mM KCl, 5mMdithioreitol, 15% v/v DMSO, 1mM de dNTP, 2mM de chaque NTP, 0,2µM d'amorces (primers), 0,1µM de torches moléculaires (molecular beacons). Cette mise en contact peut s'effectuer selon deux protocoles différents en fonction des fonctionnalités disponibles sur l'automate.

Selon un premier protocole, l'élution et la mise en condition de l'amplification se font dans deux tubes différents. Dans ce cas, il est nécessaire de prélever, dans le tube d'analyse, une fraction déterminée du volume d'élution contenant les acides nucléiques purifiés. Cette fraction est ensuite dispensée dans un second tube dans lequel va se faire la mise en condition de l'amplification et qui contient au préalable, la quantité nécessaire de tampon de mise en condition de l'amplification. Ce tube sera ensuite transféré vers l'instrument dans lequel va se dérouler l'amplification des acides nucléiques. Le prélèvement de la fraction du volume réactionnel est alors effectué par le biais de la pompe fixe 80. Dans ce cas, les vannes 70 et 82 auront été configurées de façon à permettre la communication fluidique entre la pompe 80 et l'orifice de connexion 703. Le tube d'analyse est placé en position haute de sorte que le cône trempe dans le volume d'élution. La fraction est alors aspirée par la pompe 80. Il est rappelé que cette pompe 80, et plus généralement le circuit fluidique emprunté par la fraction de volume d'élution prélevé, n'ont été en contact pendant tout le protocole de lyse et de purification qu'avec le tampon d'élution. Aucune contamination de la fraction qui va servir à l'amplification des acides nucléiques, n'est donc possible. Une fois cette fraction prélevée, le tube d'analyse peut être remplacé par le tube de mise en condition de l'amplification. A cette fin, le tube d'analyse est descendu en position basse, de manière à permettre son retrait du support et la mise en place du tube de mise en condition de l'amplification. Ce tube est alors monté en position haute de sorte que le cône pénètre dans le tube et la fraction de volume d'élution est alors dispensée par le biais de la pompe 80.

Alternativement, il peut être prévu dans l'automate, un module spécifique pour la gestion et le convoyage du tube de mise en condition de l'amplification.

Ce premier protocole présente l'avantage d'éviter que les particules de silice entre en contact avec le tampon de mise en condition de l'amplification. En effet, il est connu que de telles particules peuvent inhiber les réactions d'amplification des acides nucléiques.

Selon un second protocole, la mise en contact du tampon de mise en condition de l'amplification et du volume d'élution se fait directement dans le tube d'analyse, auquel cas, le tampon de mise en condition de l'amplification est ajouté dans le tube d'analyse. Cet ajout peut se faire soit manuellement, soit de manière automatique. Dans ce cas, l'automate doit être doté à demeure d'un second système de dispense, à savoir une récipient contenant le tampon de mise en condition de l'amplification, une seringue permettant l'aspiration et le refoulement dudit tampon, une vanne équivalente à la vanne 82 et une aiguille ou cône aptes à traversé le septum situé sur le tube d'analyse.

Quel que soit le protocole utilisé, il peut être avantageux de sortir préalablement l'amas de particules de silice à l'extérieur soit du volume d'élution seul, soit du mélange volume d'élution - tampon de mise en condition de l'amplification contenu dans le tube d'analyse, avant de prélever la fraction qui servira *in fine* pour faire l'amplification. Ceci peut être fait en soumettant le tube à une aimantation par un aimant situé en position haute.

Par ailleurs, l'ensemble des systèmes et dispositifs selon l'invention et décrits *supra* sont adaptés pour permettre une traçabilité complète tout au long des protocoles de lyse des microorganismes et de purification des acides nucléiques. En effet, en premier lieu, tous les consommables (cartouche, rotor, vanne multivoies, tubes) sont identifiés avec des moyens d'identification *ad hoc* (codes à barres 1D ou 2D, étiquettes RFID, etc...). Par ailleurs, dans le cas où la collecte de microorganismes se fait par le biais d'un moyen d'aspiration de l'air, ce dernier doit pouvoir disposer d'un moyen de communication sans fil lui permettant de communiquer avec le système de lyse de microorganismes et de purification des acides nucléiques. De tels moyens de communication sans fil sont par exemple des systèmes Wifi (norme 802.11b), Bluetooth (norme 802.15) ou ZigBee (norme 802.15.4). Les données susceptibles d'être transférées via ces systèmes de connexion sans fil peuvent être par exemple liées aux lieux (pièce/point de collecte), aux conditions de prélèvement (temps, débit, hygrométrie, température, pression atmosphérique), à l'identification de l'opérateur et bien entendu les éléments d'identification des consommables.

Les dispositif et systèmes selon l'invention est également tout à fait adapté pour l'analyse d'échantillons cliniques, tels que des échantillons de sang total ou de biopsies de tissus. Dans ce cas, la cartouche est utilisée sans module de collecte d'air. Dans le cas d'un échantillon liquide tel que du sang total, celui-ci est placé dans la cartouche puis cette dernière est combinée au rotor à la coiffe pour constituer le dispositif de lyse des microorganismes. Ce dernier est ensuite placé dans l'automate, afin d'assurer la lyse des microorganismes et la récupération des acides nucléiques. Dans le cas de biopsies de tissus, celles-ci sont préalablement découpées en tranches de quelques millimètres puis placées dans la cartouche, qui est ensuite combinée au rotor à la coiffe pour constituer le dispositif de lyse des microorganismes. Ce dernier est ensuite placé dans l'automate, afin d'assurer la lyse des bactéries et la récupération des acides nucléiques.

### EXEMPLES

### Exemple 1 : Protocole de référence :

### - Préparation des consommables :

### ∘ Préparation de la cartouche :

La cartouche est remplie avec une quantité déterminée des billes de verre et de gel d'agarose :
- 0,3 à 0,45 g de billes de verre (diamètre : 420-600 µm)
- Composition du gel : 1% agarose - 1% glycérol

Le volume de tampon d'élution utilisé pour réaliser la lyse des microorganismes est de 300µl.

### ∘ Préparation du tube d'analyse :

Le tube d'analyse placé dans l'automate est destiné à récupérer le lysat est rempli avec 30µl de particules de silice magnétique et 200µl de tampon de lyse (thiocyanate de guanidinium)

### - Paramètres de lyse :

Temps de lyse : 4 minutes
Vitesse de rotation du rotor : 200 tours/min (vitesse lente) ; 2000 tours/min (vitesse rapide).

### - Paramètres de lyse :

### Aspiration du lysat à partir de la cartouche dans la seringue de la vanne multivoies:

Volume d'aspiration de la seringue = 1000µl
Vitesse d'aspiration de la seringue= 70 mms-1
Temps de rotation du rotor pendant l'aspiration = 9 s
Vitesse de rotation du rotor pendant l'aspiration = 200 rpm

### Dispense du lysat de la seringue de la vanne multivoies vers le tube d'analyse:

Volume d'aspiration de la seringue de la vanne multivoies = 1000µl
Vitesse d'aspiration de la seringue= 70 mms-1

### - Paramètres de purification des acides nucléiques :

La purification est composée de 3 étapes :
- l'isolement des acides nucléiques
- le lavage avec le tampon d'élution (3 fois)
- l'élution dans le tampon d'élution (pH 8,5)
Après le transfert du lysat dans le tube d'analyse (0,5ml), ce dernier contient 30 µl de billes de silice, 200 µl de tampon de lyse et 120-150 µl de lysat.
Deux types d'oscillations des particules de silice sont mises en oeuvre à l'aide des aimants :
- Les oscillations hautes qui se font entre les aimants en position haute
- Les oscillations basses qui se font entre les aimants en position basse
Les oscillations hautes sont utilisées pour réaliser la capture des acides nucléiques par les particules magnétiques, mais également pour les étapes de lavage (élimination du surnageant). Les oscillations basses sont utilisées pour l'étape d'élution et sont combinées au chauffage du tube.

### Paramètres de capture des acides nucléiques :

Vitesse d'oscillation = 5 mms⁻¹
Nombre d'oscillations = 112
Amplitude d'oscillation = 27 mms⁻¹

### Paramètres de lavage :

Vitesse d'oscillation = 5 mms⁻¹
Nombre d'oscillations = 10
Amplitude d'oscillation = 27 mms⁻¹

### Paramètres d'élimination du surnageant :

Aspiration par la seringue de la vanne multivoies = 600µl
Vitesse de la pompe = 100 mms⁻¹

### Paramètres de distribution du tampon d'élution par la pompe fixe dans le tube d'analyse:

Volume de distribution = 400µl
Vitesse de la pompe= 100 mms⁻¹

### Paramètres d'élution :

Vitesse d'oscillation = 50 mms⁻¹
Amplitude d'oscillation = 9 mms⁻¹
Température cible = 75 °C
Temps de chauffage= 300 sec
Volume de tampon d'élution= 45µl

### Exemple 2 : Lyse mécanique de tissus

Une étude a été réalisée sur l'évaluation des performances de lyse mécanique dans le dispositif de lyse selon l'invention dans le but de casser les parois de cellules vivantes de types microorganismes et ainsi libérer les ARNt. Ces essais ont été menés sur des échantillons solides tumoraux.

### Protocole mis au point et testé :

### • Réactifs & matériel :

- Tumeurs de cancers du sein, pré dégraissées et stabilisées avec le matériel RNA*later*® commercialisé par Qiagen ,
- Boites de Pétri,
- Tubes 2mL stériles,
- Pinces plastiques et scalpels jetables,
- Dispositifs de lyse selon l'invention (cartouche et rotor) décontaminés à l'Actril,
- Billes de verres de 400-600µm,
- Kit RNeasy Plus commercialisé par Qiagen + automate d'extraction Qiacube.

### • Préparation :

- Nettoyer la paillasse et les pipettes avec la RNAse Zap avant de commencer l'extraction,
- Laisser décongeler les tumeurs congelées à -80°C,
- Déposer la tumeur dans une boite de pétri en évitant de prendre du RNA*later*®,
- Effectuer 2 lavages avec 300µL de tampon RLT (Qiagen) afin d'éliminer le RNAlater restant.

### • Broyage des tumeurs :

- Découpe fine de la tumeur (diamètre environ 4 mm) au scalpel,
- Récupération des morceaux de tumeur à l'aide d'une pince plastique et transfert dans un tube de 2mL contenant 300µL de RLT (+3µL de β-mercaptoéthanol),
- Broyage les morceaux de tumeur avec :
   - le Tissue Ruptor Qiagen : vitesse 2 pendant 2 minutes, ou
      le dispositif de lyse de microorganismes selon l'invention : 0,4-0,5 gr de billes de verre (400-600µm), 2000 tr/min, 2 fois 20 secondes.
- Récupération d'échantillon et ajustement du volume avec le tampon RLT pour avoir un volume 300µL avant la purification.

### • Purification des ARNt avec le kit Qiagen RNeasy Plus Mini :

1. Mettre les 300µL de tampon RLT récupérés après broyage sans les débris de tumeurs restants dans un tube de 2mL,
2. Préparer les 2 types de colonnes pour la purification et la fixation des ARN : colonne gDNA Eliminator et RNA spin,
3. Préparer les tampons, les navettes d'extraction Qiagen ainsi que les tubes d'élution,
4. Préparer l'essai Qiacube en suivant les instruction édictées par le robot et lancer le run,
5. Récupérer les 80µl d'éluat et congeler à -80°C les ARNt purifiés,
6. Dosage : 1µL d'éluat non dilué au Nanodrop,
7. Mesure de la qualité de l'ARNt extrait : l'analyse de la qualité est réalisée selon le protocole RNA 6000 Nano sur bioanalyseur Agilent.

De même que dans une électrophorèse classique on observe la répartition des transcrits avec deux pics principaux pour les ARNr 18s et 28s.

### Résultats :

Deux biopsies ont été utilisées pour cette étude, toutes préparées et conservées de manière identique. Ces biopsies sont circulaires, d'un diamètre d'environ 4 mm et d'épaisseur 1,5 mm. Leur masse est évaluée approximativement à un dizaine de mg. Ces biopsies ont été grossièrement partagées en deux afin de mener en parallèle les deux types de lyse mécanique (lyse de référence Tissu Ruptor et PPM). Il est toutefois à noter que la quantité de tissu utilisé n'est pas identique d'un essai à l'autre, le but étant de s'assurer du bon déroulement de l'essai, plutôt que de faire une comparaison de performances entre la technique de référence et la protocole selon l'invention.

L'analyse de la pureté est obtenue par ration entre les valeurs d'absorbance à différentes longueurs d'onde : 260nm pour les acides nucléiques, 230nm pour les sels et 280nm pour les protéines. Une valeur de ratio proche de 2 reflète une bonne pureté des acides nucléiques.

| **Biopsie** | **Protocole Extraction** | | **ARNt** (ng/µl) | **Pureté** | |
|---|---|---|---|---|---|
| | Lyse | Purification | | 260/280 | 260/230 |
| **1** | Tissue ruptor (Qiagen) 2 min, vitesse 2 | Kit : RNeasy plus | 3,4 | 0,86 | 0,19 |
| | | Robot : Qiacube | | | |
| **1** | **Dispositif selon l'invention 2x20 sec à 2000 tr/min 0,4 gr de billes 420-600 µm** | **Kit : RNeasy plus** | **2,2** | **0,42** | **0,11** |
| | | **Robot : Qiacube** | | | |
| **2** | Tissue ruptor de Qiagen 2 min, vitesse 2 | Kit : RNeasy plus | 31,7 | 2,09 | 0,56 |
| | | Robot : Qiacube | | | |
| **2** | **Dispositif selon l'invention 2x20 sec à 2000 tr/min 0,4 gr de billes 420-600 µm** | **Kit : RNeasy plus** | **81,1** | **2,10** | **1,70** |
| | | **Robot : Qiacube** | | | |

Dans le cas de la biopsie 1, on constate que la quantité d'ARNt extraite est très faible. Ceci peut s'expliquer par le fait que la biopsie s'est dégradée, entraînant une perte de matériel.

Dans le cas de la biopsie 2, on constate qu'une quantité significative d'ARNt a été extraite par le deux techniques. On constate par ailleurs que la pureté est bonne.

Il ressort ainsi de ces exemples que le dispositif selon l'invention est un outil performant pour collecter les bactéries contenues dans un échantillon d'air, les lyser et permettre la récupération des acides nucléiques desdites bactéries à fins d'analyse. Pour cela, le dispositif mis en oeuvre sera constitué dans un premier temps d'une cartouche insérée à l'intérieur du module de collecte d'air et, dans un deuxième temps, de la cartouche contenant les bactéries collectées, combinée au moyen de récupération des acides nucléiques.

## Revendications

1. Dispositif de collecte de microorganismes contenus dans l'air, ledit dispositif comportant :
- un module de collecte d'air, comprenant :
i. un élément supérieur comportant un conduit d'admission de l'air permettant l'entrée d'un flux d'air à l'intérieur dudit module, ledit conduit disposant à sa base, de moyens de perturbation du flux d'air,
ii. un élément inférieur comportant des moyens d'évacuation de l'air permettant la sortie du flux d'air créé
lesdits éléments supérieur et inférieur pouvant être solidarisés l'un à l'autre de sorte que le flux d'air puisse être créé à l'intérieur dudit module de collecte d'air ;
- une cartouche, de forme sensiblement cylindrique, comportant une zone de rétention des microorganismes, ladite zone de rétention comportant des moyens de lyse de microorganismes, ladite cartouche étant positionnée à l'intérieur dudit module de collecte d'air.

2. Dispositif selon la revendication précédente, dans lequel la zone de rétention des microorganismes de la cartouche comporte, en outre, un matériau apte à retenir les microorganismes, à maintenir les moyens de lyse en place et à se dissoudre en présence d'un liquide.

3. Dispositif selon la revendication 1 ou 2, dans lequel les moyens de perturbation du flux d'air comportent un cône 30 en forme d'ogive positionné au centre de la lumière du conduit d'admission de l'air et au moins une ailette reliant ladite pièce en ogive à la face interne du conduit d'admission d'air.

4. Dispositif selon l'une des revendications précédentes, dans lequel le module de collecte d'air est destiné à être connecté à un circuit de recyclage de l'air ou à un dispositif d'aspiration de l'air.

5. Dispositif de lyse de microorganismes, ledit dispositif comportant :
- une cartouche, de forme sensiblement cylindrique, comportant une zone de rétention des microorganismes, ladite zone de rétention comportant des moyens de lyse de microorganismes et un matériau apte à retenir lesdits microorganismes, à maintenir les moyens de lyse en place et à se dissoudre en présence de liquide, ladite cartouche jouant le rôle de stator ;
- un rotor, apte à être placé dans la cartouche, ledit rotor comportant des moyens de mise en rotation.

6. Dispositif selon la revendication précédente, comportant en outre une coiffe destinée à maintenir le rotor solidaire de la cartouche.

7. Dispositif selon la revendication 5 ou 6, dans lequel les moyens de mise en rotation sont constitués par des rainures ménagées dans la paroi d'un conduit borgne, lesdites rainures étant diamétralement opposées et destinés à recevoir l'extrémité d'un axe de transmission d'un moyen d'entraînement dudit rotor en rotation.

8. Dispositif selon l'une des revendications 5 à 7, dans lequel le diamètre interne de la cartouche est supérieur au diamètre externe du rotor, de sorte que lorsque le rotor est emboîté dans la cartouche, la distance séparant la paroi interne de la cartouche de la paroi externe du rotor est suffisamment importante pour permettre aux moyens de lyse de venir se positionner dans cet espace interstitiel et suffisamment faible pour que les moyens de lyse soient au contact de l'une ou l'autre desdites parois.

9. Système de lyse de microorganismes et de purification des acides nucléiques desdits microorganismes, comportant :
- un moyen de positionnement du dispositif de lyse de microorganismes selon l'une des revendication 5 à 8 ; un dispositif de lyse de microorganismes selon l'une des revendications 5à 8;
- un moyen d'entraînement du rotor en rotation, lorsque le dispositif de lyse est placé sur le moyen de positionnement ;
- au moins un conteneur de réception des acides nucléiques ;
- au moins un moyen d'aspiration/refoulement de liquide ;
- une vanne multivoies, en communication fluidique avec le dispositif de lyse de microorganismes, conteneur de réception des acides nucléiques purifiés et le moyen d'aspiration/refoulement de liquide.

10. Système de lyse selon la revendication précédente, comportant en outre des moyens d'aimantation.

11. Système de lyse selon la revendication 9 ou 10, comportant en outre des moyens de chauffage.

12. Système de lyse selon l'une des revendications 9 à 11, dans lequel la vanne multivoies comporte un moyen d'aspiration-refoulement.

13. Procédé de collecte des microorganismes contenus dans l'air, ledit procédé comprenant les étapes consistant à :
a) Placer une cartouche selon les revendications 1 ou 2, à l'intérieur d'un module de collecte d'air, selon les revendications 1 ou 3, de sorte d'obtenir le dispositif de collecte de microorganismes selon la revendication 1 à 3, la zone de rétention à l'intérieur de ladite cartouche étant en communication avec le conduit d'admission d'air du module de collecte d'air,
b) Provoquer l'entrée d'air à l'intérieur dudit module de collecte d'air par tout moyen approprié,
c) Collecter les microorganismes contenus dans l'air dans la zone de rétention de la cartouche.

14. Procédé de lyse de microorganismes contenus dans l'air, ledit procédé comportant les étapes consistant à :
a) Placer une cartouche selon les revendications 1 ou 2, à l'intérieur d'un module de collecte selon les revendications 1 ou 3, de sorte d'obtenir le dispositif de collecte de microorganismes selon la revendication 1 à 3, la zone de rétention à l'intérieur de ladite cartouche étant en communication avec le conduit d'admission d'air du module de collecte d'air,
b) Provoquer l'entrée d'air à l'intérieur dudit module de collecte d'air par tout moyen approprié,
c) Collecter les microorganismes contenus dans l'air, dans la zone de rétention de la cartouche,
d) Sortir la cartouche du module de collecte d'air,
e) Placer le rotor dans la cartouche, de sorte d'obtenir le dispositif de lyse de microorganismes selon l'une des revendications 5 à 8,
f) Positionner le dispositif de lyse de microorganismes dans le système de lyse de microorganismes et de purification des acides nucléiques,
g) Introduire un liquide d'élution dans la cartouche, entraînant la mise en suspension des moyens de lyse situés dans la zone de rétention des microorganismes de la cartouche, et
h) Lyser mécaniquement les microorganismes, par mise en rotation du rotor à l'intérieur de la cartouche grâce au moyen d'entraînement en rotation dudit rotor, ledit rotor entraînant en rotation les moyens de lyse des microorganismes.

15. Procédé d'extraction des acides nucléiques de microorganismes contenus dans l'air, ledit procédé comportant les étapes consistant à :
a) Placer une cartouche selon les revendications 1 ou 2, à l'intérieur d'un module de collecte d'air, selon les revendications 1 ou 3, de sorte d'obtenir le dispositif de collecte de microorganismes selon l'une des revendications 1 à 3, la zone de rétention à l'intérieur de ladite cartouche étant en communication avec le conduit d'admission d'air du module de collecte d'air,
b) Provoquer l'entrée d'air à l'intérieur dudit module de collecte d'air par tout moyen approprié,
c) Collecter les microorganismes contenus dans l'air, dans la zone de rétention de la cartouche,
d) Sortir la cartouche du module de collecte d'air,
e) Placer le rotor selon les revendications 5-8, la cartouche, selon les revendication 5-8, de sorte d'obtenir le dispositif de lyse de microorganismes selon l'une des revendications 5 à 8,
f) Introduire un liquide d'élution dans la cartouche à l'aide d'un moyen d'aspiration - refoulement, entraînant la mise en suspension des moyens de lyse contenus dans la cartouche, et
g) Lyser mécaniquement les microorganismes, par mise en rotation du rotor, à l'intérieur de la cartouche grâce au moyen d'entraînement en rotation dudit rotor, ledit rotor entraînant en rotation les moyens de lyse, et
h) Aspirer le liquide d'élution contenant les acides nucléiques desdits microorganismes libérés lors de la lyse, et
i) Dispenser le liquide d'élution dans un conteneur de réception et purification des acides nucléiques.

16. Procédé selon la revendication précédente, comprenant une étape supplémentaire de lavage et de purification des acides nucléiques.

17. Procédé selon la revendication 13 à 16, comportant une étape supplémentaire d') consistant à faire croître les microorganismes concentrés dans la zone de rétention de la cartouche.

18. Procédé selon la revendication précédente, dans lequel la mise en croissance est obtenue par incubation de la cartouche dans une étuve pendant un temps allant de 2 à 24 heures.

19. Procédé selon l'une des revendications 15 à 18, dans lequel les étapes f) à i) sont mises en oeuvre dans le système de lyse des microorganismes et de purification des acides nucléiques selon l'une des revendications 9 à 12.

20. Procédé de lyse de microorganismes, ledit procédé comportant les étapes consistant à :
a) Obtenir une cartouche dans laquelle des microorganismes sont concentrés dans la zone de rétention de ladite cartouche,
b) Placer le rotor selon les revendications 5-8, dans la cartouche, selon les revendications 5-8 de sorte d'obtenir le dispositif de lyse de microorganismes selon l'une des revendications 5 à 8,
c) Introduire un liquide d'élution dans la cartouche, entraînant la mise en suspension des moyens de lyse situés dans la zone de rétention des microorganismes de la cartouche, et
d) Lyser mécaniquement les microorganismes, par mise en rotation du rotor, à l'intérieur de la cartouche, ledit rotor entraînant en rotation les moyens de lyse des microorganismes.

21. Procédé d'extraction des acides nucléiques de microorganismes, ledit procédé comportant les étapes consistant à :
a) Obtenir une cartouche dans laquelle des microorganismes sont concentrés dans la zone de rétention,
b) Placer le rotor selon les revendications 5-8 dans la selon les revendications 5-8, de sorte d'obtenir le dispositif de lyse de microorganismes selon l'une des revendications 5 à 8,
c) Introduire un liquide d'élution dans la cartouche, entraînant la mise en suspension des moyens de lyse situés dans la zone de rétention des microorganismes de la cartouche, et
d) Lyser mécaniquement les microorganismes, par mise en rotation du rotor, à l'intérieur de la cartouche, ledit rotor entraînant en rotation les moyens de lyse des microorganismes, et
e) Aspirer le liquide d'élution contenant les acides nucléiques desdits microorganismes libérés lors de la lyse, et
f) Dispenser le liquide d'élution dans un conteneur de réception et de purification des acides nucléiques.

22. Procédé selon la revendication précédente, comprenant une étape supplémentaire de lavage et de purification des acides nucléiques.

23. Procédé de lyse de microorganismes contenus dans un échantillon liquide, ledit procédé comportant les étapes consistant à :
a) Introduire l'échantillon liquide contenant lesdits microorganismes dans une cartouche, au voisinage de la zone de rétention, de sorte que ledit échantillon liquide entraîne la mise en suspension des moyens de lyse situés dans ladite zone de rétention des microorganismes de la cartouche,
b) Placer le rotor dans la cartouche, de sorte d'obtenir le dispositif de lyse de microorganismes selon l'une des revendications 5 à 8,
c) Lyser mécaniquement les microorganismes, par mise en rotation du rotor, à l'intérieur de la cartouche, ledit rotor entraînant en rotation les moyens de lyse sur lesquels sont retenus les microorganismes.

24. Procédé d'extraction des acides nucléiques de microorganismes contenus dans un échantillon liquide, ledit procédé comportant les étapes consistant à :
a) Introduire un échantillon liquide contenant lesdits microorganismes dans une cartouche au voisinage de la zone de rétention, de sorte que ledit échantillon liquide entraîne la mise en suspension des moyens de lyse situés dans ladite zone de rétention des microorganismes de la cartouche,
b) Placer le rotor selon les revendications 5-8, dans la cartouche, selon les revendications 5-8, de sorte d'obtenir le dispositif de lyse de microorganismes selon l'une des revendications 5 à 8,
c) Lyser mécaniquement les microorganismes, par mise en rotation du rotor, à l'intérieur de la cartouche, ledit rotor entraînant en rotation les moyens de lyse,
d) Aspirer l'échantillon liquide contenant les acides nucléiques desdits microorganismes libérés lors de la lyse et
e) Dispenser l'échantillon liquide dans un conteneur de réception et de purification des acides nucléiques.

25. Procédé d'extraction des acides nucléiques d'un échantillon de tissu cellulaire, ledit procédé comportant les étapes consistant à :
a) Introduire l'échantillon de tissu cellulaire dans la cartouche en présence d'un liquide d'élution,
b) Placer le rotor selon les revendications 5-8, dans la cartouche, selon les revendications 5-8,
c) Lyser mécaniquement les cellules du tissu cellulaire, par mise en rotation du rotor, à l'intérieur de la cartouche, ledit rotor entraînant en rotation les moyens de lyse contenus dans la cartouche,
d) Aspirer le liquide d'élution contenant les acides nucléiques desdits cellules, libérés lors de la lyse et
e) Dispenser le liquide d'élution dans un conteneur de réception et de purification des acides nucléiques.

26. Procédé selon l'une des revendications 24 ou 25, comprenant une étape supplémentaire de lavage et de purification des acides nucléiques.

27. Utilisation du dispositif de lyse de microorganismes selon l'une des revendications 5 à 8, pour lyser des cellules d'un échantillon de tissu cellulaire.

28. Utilisation du système de lyse de microorganismes et de purification des acides nucléiques desdits microorganismes selon l'une des revendication 9 à 12, pour lyser des cellules d'un échantillon de tissu cellulaire.

## Patentansprüche

1. Vorrichtung zum Sammeln von in der Luft enthaltenen Mikroorganismen, wobei die Vorrichtung aufweist:
- ein Luftsammelmodul, umfassend:
i. ein oberes Element, das einen Lufteinlasskanal aufweist, der den Eintritt eines Luftstroms ins Innere des Moduls ermöglicht, wobei der Kanal an seinem Fuß über Mittel zur Störung des Luftstroms verfügt,
ii. ein unteres Element, das Luftauslassmittel aufweist, die den Austritt des erzeugten Luftstroms ermöglichen,
wobei das obere und das untere Element fest miteinander verbunden werden können, derart, dass der Luftstrom im Inneren des Luftsammelmoduls erzeugt werden kann;
- eine Kartusche von im Wesentlichen zylindrischer Form, die einen Bereich zum Zurückhalten der Mikroorganismen aufweist, wobei der Zurückhaltebereich Mittel zur Lyse von Mikroorganismen aufweist, wobei die Kartusche im Inneren des Luftsammelmoduls positioniert ist.

2. Vorrichtung nach dem vorhergehenden Anspruch, wobei der Bereich zum Zurückhalten der Mikroorganismen der Kartusche außerdem ein Material aufweist, das geeignet ist, die Mikroorganismen zurückzuhalten, die Lysemittel in Position zu halten und sich in Gegenwart einer Flüssigkeit aufzulösen.

3. Vorrichtung nach Anspruch 1 oder 2, wobei die Mittel zur Störung des Luftstroms einen spitzbogenförmigen Kegel 30, der in der Mitte des Hohlraums des Lufteinlasskanals positioniert ist, und wenigstens eine Rippe, die das spitzbogenförmige Teil mit der Innenfläche des Lufteinlasskanals verbindet, aufweisen.

4. Vorrichtung nach einem der vorhergehenden Ansprüche, wobei das Luftsammelmodul dazu bestimmt ist, mit einem Luftrückführungskreislauf oder mit einer Luftansaugvorrichtung verbunden zu werden.

5. Vorrichtung zur Lyse von Mikroorganismen, wobei die Vorrichtung aufweist:
- eine Kartusche von im Wesentlichen zylindrischer Form, die einen Bereich zum Zurückhalten der Mikroorganismen aufweist, wobei der Zurückhaltebereich Mittel zur Lyse von Mikroorganismen und ein Material, das geeignet ist, die Mikroorganismen zurückzuhalten, die Lysemittel in Position zu halten und sich in Gegenwart von Flüssigkeit aufzulösen, aufweist, wobei die Kartusche die Funktion eines Stators erfüllt;
- einen Rotor, der geeignet ist, in der Kartusche angebracht zu werden, wobei der Rotor Mittel zum Versetzen in Drehung aufweist.

6. Vorrichtung nach dem vorhergehenden Anspruch, welche außerdem eine Kappe aufweist, die dazu bestimmt ist, den Rotor fest mit der Kartusche verbunden zu halten.

7. Vorrichtung nach Anspruch 5 oder 6, wobei die Mittel zum Versetzen in Drehung aus Rillen bestehen, die in der Wand eines einseitig geschlossenen Kanals ausgebildet sind, wobei die Rillen einander diametral gegenüberliegen und dazu bestimmt sind, das Ende einer Kraftübertragungsachse eines Mittels zum Drehantrieb des Rotors aufzunehmen.

8. Vorrichtung nach einem der Ansprüche 5 bis 7, wobei der Innendurchmesser der Kartusche größer als der Außendurchmesser des Rotors ist, derart, dass, wenn der Rotor in die Kartusche eingefügt ist, der Abstand, der die Innenwand der Kartusche von der Außenwand des Rotors trennt, genügend groß ist, um zu ermöglichen, dass die Lysemittel in diesem Zwischenraum positioniert werden, und genügend klein dafür, dass sich die Lysemittel in Kontakt mit der einen oder der anderen der Wände befinden.

9. System zur Lyse von Mikroorganismen und Reinigung der Nukleinsäuren der Mikroorganismen, welches aufweist:
- ein Mittel zur Positionierung der Vorrichtung zur Lyse von Mikroorganismen nach einem der Ansprüche 5 bis 8;
- eine Vorrichtung zur Lyse von Mikroorganismen nach einem der Ansprüche 5 bis 8;
- ein Mittel zum Drehantrieb des Rotors, wenn die Lysevorrichtung auf dem Positionierungsmittel angebracht ist;
- wenigstens einen Behälter zur Aufnahme der Nukleinsäuren;
- wenigstens ein Mittel zum Ansaugen/Fördern von Flüssigkeit;
- ein Mehrwegeventil, das mit der Vorrichtung zur Lyse von Mikroorganismen, dem Behälter zur Aufnahme der gereinigten Nukleinsäuren und dem Mittel zum Ansaugen/Fördern von Flüssigkeit in Fluidverbindung steht.

10. Lysesystem nach dem vorhergehenden Anspruch, welches außerdem Magnetisierungsmittel aufweist.

11. Lysesystem nach Anspruch 9 oder 10, welches außerdem Heizmittel aufweist.

12. Lysesystem nach einem der Ansprüche 9 bis 11, wobei das Mehrwegeventil ein Ansaug-/Fördermittel aufweist.

13. Verfahren zum Sammeln der in der Luft enthaltenen Mikroorganismen, wobei das Verfahren die folgenden Schritte umfasst:
a) Anbringen einer Kartusche nach den Ansprüchen 1 oder 2 im Inneren eines Luftsammelmoduls nach den Ansprüchen 1 oder 3, um die Vorrichtung zum Sammeln von Mikroorganismen nach Anspruch 1 bis 3 zu erhalten, wobei der Zurückhaltebereich im Inneren der Kartusche mit dem Lufteinlasskanal des Luftsammelmoduls in Verbindung steht,
b) Hervorrufen des Eintritts von Luft ins Innere des Luftsammelmoduls mit einem beliebigen geeigneten Mittel,
c) Sammeln der in der Luft enthaltenen Mikroorganismen in dem Zurückhaltebereich der Kartusche.

14. Verfahren zur Lyse von in der Luft enthaltenen Mikroorganismen, wobei das Verfahren die folgenden Schritte umfasst:
a) Anbringen einer Kartusche nach den Ansprüchen 1 oder 2 im Inneren eines Sammelmoduls nach den Ansprüchen 1 oder 3, um die Vorrichtung zum Sammeln von Mikroorganismen nach Anspruch 1 bis 3 zu erhalten, wobei der Zurückhaltebereich im Inneren der Kartusche mit dem Lufteinlasskanal des Luftsammelmoduls in Verbindung steht,
b) Hervorrufen des Eintritts von Luft ins Innere des Luftsammelmoduls mit einem beliebigen geeigneten Mittel,
c) Sammeln der in der Luft enthaltenen Mikroorganismen in dem Zurückhaltebereich der Kartusche,
d) Entnehmen der Kartusche aus dem Luftsammelmodul,
e) Anbringen des Rotors in der Kartusche, derart, dass die Vorrichtung zur Lyse von Mikroorganismen nach einem der Ansprüche 5 bis 8 erhalten wird,
f) Positionieren der Vorrichtung zur Lyse von Mikroorganismen in dem System zur Lyse von Mikroorganismen und Reinigung der Nukleinsäuren,
g) Einleiten einer Elutionsflüssigkeit in die Kartusche, was die Suspendierung der Lysemittel zur Folge hat, die sich in dem Bereich zum Zurückhalten der Mikroorganismen der Kartusche befinden, und
h) mechanisches Lysieren der Mikroorganismen durch Versetzen des Rotors in Drehung im Inneren der Kartusche mithilfe des Mittels zum Drehantrieb des Rotors, wobei der Rotor die Mittel zur Lyse der Mikroorganismen drehend antreibt.

15. Verfahren zur Extraktion der Nukleinsäuren von in der Luft enthaltenen Mikroorganismen, wobei das Verfahren die folgenden Schritte umfasst:
a) Anbringen einer Kartusche nach den Ansprüchen 1 oder 2 im Inneren eines Luftsammelmoduls nach den Ansprüchen 1, oder 3, um die Vorrichtung zum Sammeln von Mikroorganismen nach einem der Ansprüche 1 bis 3 zu erhalten, wobei der Zurückhaltebereich im Inneren der Kartusche mit dem Lufteinlasskanal des Luftsammelmoduls in Verbindung steht,
b) Hervorrufen des Eintritts von Luft ins Innere des Luftsammelmoduls mit einem beliebigen geeigneten Mittel,
c) Sammeln der in der Luft enthaltenen Mikroorganismen in dem Zurückhaltebereich der Kartusche,
d) Entnehmen der Kartusche aus dem Luftsammelmodul,
e) Anbringen des Rotors nach den Ansprüchen 5-8, der Kartusche nach den Ansprüchen 5-8, um die Vorrichtung zur Lyse von Mikroorganismen nach einem der Ansprüche 5 bis 8 zu erhalten,
f) Einleiten einer Elutionsflüssigkeit in die Kartusche mithilfe eines Ansaug-/Fördermittels, was die Suspendierung der in der Kartusche enthaltenen Lysemittel zur Folge hat, und
g) mechanisches Lysieren der Mikroorganismen durch Versetzen des Rotors in Drehung im Inneren der Kartusche mithilfe des Mittels zum Drehantrieb des Rotors, wobei der Rotor die Lysemittel drehend antreibt, und
h) Ansaugen der Elutionsflüssigkeit, welche die bei der Lyse freigesetzten Nukleinsäuren der Mikroorganismen enthält, und
i) Abgeben der Elutionsflüssigkeit in einen Behälter zur Aufnahme und Reinigung der Nukleinsäuren.

16. Verfahren nach dem vorhergehenden Anspruch, welches einen zusätzlichen Schritt des Waschens und der Reinigung der Nukleinsäuren umfasst.

17. Verfahren nach Anspruch 13 bis 16, welches einen zusätzlichen Schritt d') umfasst, der darin besteht, die in dem Zurückhaltebexeich der Kartusche konzentrierten Mikroorganismen wachsen zu lassen.

18. Verfahren nach dem vorhergehenden Anspruch, wobei das Wachstum durch Inkubation der Kartusche in einem Wärmeschrank während eines Zeitraums von 2 bis 24 Stunden erzielt wird.

19. Verfahren nach einem der Ansprüche 15 bis 18, wobei die Schritte f) bis i) in dem System zur Lyse der Mikroorganismen und Reinigung der Nukleinsäuren nach einem der Ansprüche 9 bis 12 durchgeführt werden.

20. Verfahren zur Lyse von Mikroorganismen, wobei das Verfahren die folgenden Schritte umfasst:
a) Erhalten einer Kartusche, in welcher Mikroorganismen in dem Zurückhaltebereich der Kartusche konzentriert sind,
b) Anbringen des Rotors nach den Ansprüchen 5-8 in der Kartusche nach den Ansprüchen 5-8, um die Vorrichtung zur Lyse von Mikroorganismen nach einem der Ansprüche 5 bis 8 zu erhalten,
c) Einleiten einer Elutionsflüssigkeit in die Kartusche, was die Suspendierung der Lysemittel zur Folge hat, die sich in dem Bereich zum Zurückhalten der Mikroorganismen der Kartusche befinden, und
d) mechanisches Lysieren der Mikroorganismen durch Versetzen des Rotors in Drehung im Inneren der Kartusche, wobei der Rotor die Mittel zur Lyse der Mikroorganismen drehend antreibt.

21. Verfahren zur Extraktion der Nukleinsäuren von Mikroorganismen, wobei das Verfahren die folgenden Schritte umfasst:
a) Erhalten einer Kartusche, in welcher Mikroorganismen in dem Zurückhaltebereich konzentriert sind,
b) Anbringen des Rotors nach den Ansprüchen 5-8 in der Kartusche nach den Ansprüchen 5-8, um die Vorrichtung zur Lyse von Mikroorganismen nach einem der Ansprüche 5 bis 8 zu erhalten,
c) Einleiten einer Elutionsflüssigkeit in die Kartusche, was die Suspendierung der Lysemittel zur Folge hat, die sich in dem Bereich zum Zurückhalten der Mikroorganismen der Kartusche befinden, und
d) mechanisches Lysieren der Mikroorganismen durch Versetzen des Rotors in Drehung im Inneren der Kartusche, wobei der Rotor die Mittel zur Lyse der Mikroorganismen drehend antreibt, und
e) Ansaugen der Elutionsflüssigkeit, welche die bei der Lyse freigesetzten Nukleinsäuren der Mikroorganismen enthält, und
f) Abgeben der Elutionsflüssigkeit in einen Behälter zur Aufnahme und Reinigung der Nukleinsäuren.

22. Verfahren nach dem vorhergehenden Anspruch, welches einen zusätzlichen Schritt des Waschens und der Reinigung der Nukleinsäuren umfasst.

23. Verfahren zur Lyse von in einer flüssigen Probe enthaltenen Mikroorganismen, wobei das Verfahren die folgenden Schritte umfasst:
a) Einleiten der die Mikroorganismen enthaltenden flüssigen Probe in eine Kartusche in der Nähe des Zurückhaltebereichs, derart, dass die flüssige Probe die Suspendierung der Lysemittel bewirkt, die sich in dem Bereich zum Zurückhalten der Mikroorganismen der Kartusche befinden,
b) Anbringen des Rotors in der Kartusche, um die Vorrichtung zur Lyse von Mikroorganismen nach einem der Ansprüche 5 bis 8 zu erhalten,
c) mechanisches Lysieren der Mikroorganismen durch Versetzen des Rotors in Drehung im Inneren der Kartusche, wobei der Rotor die Lysemittel, auf denen die Mikroorganismen zurückgehalten werden, drehend antreibt.

24. Verfahren zur Extraktion der Nukleinsäuren von in einer flüssigen Probe enthaltenen Mikroorganismen, wobei das Verfahren die folgenden Schritte umfasst:
a) Einleiten einer die Mikroorganismen enthaltenden flüssigen Probe in eine Kartusche in der Nähe des Zurückhaltebereichs, derart, dass die flüssige Probe die Suspendierung der Lysemittel bewirkt, die sich in dem Bereich zum Zurückhalten der Mikroorganismen der Kartusche befinden,
b) Anbringen des Rotors nach den Ansprüchen 5-8 in der Kartusche nach den Ansprüchen 5-8, um die Vorrichtung zur Lyse von Mikroorganismen nach einem der Ansprüche 5 bis 8 zu erhalten,
c) mechanisches Lysieren der Mikroorganismen durch Versetzen des Rotors in Drehung im Inneren der Kartusche, wobei der Rotor die Lysemittel drehend antreibt,
d) Ansaugen der flüssigen Probe, welche die bei der Lyse freigesetzten Nukleinsäuren der Mikroorganismen enthält, und
e) Abgeben der flüssigen Probe in einen Behälter zur Aufnahme und Reinigung der Nukleinsäuren.

25. Verfahren zur Extraktion der Nukleinsäuren einer Zellgewebeprobe, wobei das Verfahren die folgenden Schritte umfasst:
a) Einführen der Zellgewebeprobe in die Kartusche in Gegenwart einer Elutionsflüssigkeit,
b) Anbringen des Rotors nach den Ansprüchen 5-8 in der Kartusche nach den Ansprüchen 5-8,
c) mechanisches Lysieren der Zellen des Zellgewebes durch Versetzen des Rotors in Drehung im Inneren der Kartusche, wobei der Rotor die in der Kartusche enthaltenen Lysemittel drehend antreibt,
d) Ansaugen der Elutionsflüssigkeit, welche die bei der Lyse freigesetzten Nukleinsäuren der Zellen enthält, und
e) Abgeben der Elutionsflüssigkeit in einen Behälter zur Aufnahme und Reinigung der Nukleinsäuren.

26. Verfahren nach einem der Ansprüche 24 oder 25, welches einen zusätzlichen Schritt des Waschens und der Reinigung der Nukleinsäuren umfasst.

27. Verwendung der Vorrichtung zur Lyse von Mikroorganismen nach einem der Ansprüche 5 bis 8 zum Lysieren der Zellen einer Zellgewebeprobe.

28. Verwendung des Systems zur Lyse von Mikroorganismen und Reinigung der Nukleinsäuren der Mikroorganismen nach einem der Ansprüche 9 bis 12 zum Lysieren der Zellen einer Zellgewebeprobe.

## Claims

1. A device for collecting airborne microorganisms, said device having:
- an air collecting module, comprising:
i. an upper element having an air admission duct permitting entry of an air stream into said module, said duct being provided, at its base, with means for disturbance of the air stream,
ii. a lower element having means for evacuation of the air, permitting the air stream created to exit
and said upper and lower elements can be made integral with one another so that the air stream can be created within said air collecting module;
- a cartridge, of roughly cylindrical shape, having a microorganism retention zone, said retention zone having means for lysis of the microorganisms, said cartridge being positioned within said air collecting module.

2. The device as claimed in the preceding claim, in which the microorganism retention zone of the cartridge further comprises a material that is able to retain the microorganisms, to hold the means for lysis in place and to be dissolved in the presence of a liquid.

3. The device as claimed in claim 1 or 2, in which the means for disturbance of the air stream comprises an ogive-shaped cone 30 positioned at the center of the bore of the air admission duct and at least one blade connecting said ogive-shaped piece to the inside surface of the air admission duct.

4. The device as claimed in one of the preceding claims, in which the air collecting module is intended to be connected to an air recycling circuit or to an air aspirating device.

5. A device for lysis of microorganisms, said device having:
- a cartridge, of roughly cylindrical shape, having a microorganism retention zone, said retention zone having means for lysis of the microorganisms and a material that is able to retain the microorganisms, to hold the means for lysis in place and to be dissolved in the presence of a liquid, said cartridge performing the role of stator;
- a rotor, which can be placed in the cartridge, said rotor having rotating means.

6. The device as claimed in the preceding claim, having in addition a cap intended for keeping the rotor integral with the cartridge.

7. The device as claimed in claim 5 or 6, in which the rotating means is constituted of grooves made in the wall of a blind duct, said grooves being diametrically opposite and intended to receive the end of a transmission shaft of a means for rotating said rotor.

8. The device as claimed in one of claims 5 to 7, in which the inside diameter of the cartridge is greater than the outside diameter of the rotor, so that when the rotor is fitted in the cartridge, the distance from the inside wall of the cartridge to the outside wall of the rotor is large enough to permit the means for lysis to be positioned in this interstitial space and small enough for the means for lysis to be in contact with one or other of said walls.

9. A system for lysis of microorganisms and purification of the nucleic acids of said microorganisms, having:
- means for positioning the device for lysis of microorganisms as claimed in one of claims 5 to 8;
- means for rotating the rotor, when the lysis device is placed on the positioning means;
- at least one container for receiving the nucleic acids;
- at least one means for aspiration/discharge of liquid;
- a multi-way valve, in fluidic communication with the device for lysis of microorganisms, container for receiving purified nucleic acids and means for aspiration/discharge of liquid.

10. The lysis system as claimed in the preceding claim, having in addition means for magnetization.

11. The lysis system as claimed in claim 9 or 10, having in addition heating means.

12. The lysis system as claimed in one of claims 9 to 11, in which the multi-way valve comprises aspiration-discharge means.

13. A method of collecting airborne microorganisms, said method comprising stages consisting of:
a) placing a cartridge inside an air collecting module, so as to obtain the microorganism collecting device as claimed in claims 1 to 3, the retention zone within said cartridge being in communication with the air admission duct of the air collecting module,
b) causing air to go into said air collecting module by any suitable means,
c) collecting the airborne microorganisms in the retention zone of the cartridge.

14. A method of lysis of airborne microorganisms, said method having stages consisting of:
a) placing a cartridge inside an air collecting module, so as to obtain the microorganism collecting device as claimed in claim 1 to 3, the retention zone within said cartridge being in communication with the air admission duct of the air collecting module,
b) causing air to go into said air collecting module by any suitable means,
c) collecting the airborne microorganisms, in the retention zone of the cartridge,
d) removing the cartridge from the air collecting module,
e) placing the rotor in the cartridge, so as to obtain the device for lysis of microorganisms as claimed in one of claims 5 to 8,
f) positioning the device for lysis of microorganisms in the system for lysis of microorganisms and purification of nucleic acids,
g) introducing an elution liquid into the cartridge, for suspending the lysis means situated in the microorganism retention zone of the cartridge, and
h) mechanically lysing the microorganisms, by rotating the rotor within the cartridge by the rotating means of said rotor, said rotor causing the means for lysis of microorganisms to rotate.

15. A method of extracting the nucleic acids from airborne microorganisms, said method having stages consisting of:
a) placing a cartridge inside an air collecting module, so as to obtain the microorganism collecting device as claimed in one of claims 1 to 3, the retention zone within said cartridge being in communication with the air admission duct of the air collecting module,
b) causing air to go into said air collecting module by any suitable means,
c) collecting the airborne microorganisms, in the retention zone of the cartridge,
d) removing the cartridge from the air collecting module,
e) placing the rotor in the cartridge, so as to obtain the device for lysis of microorganisms as claimed in one of claims 5 to 8,
f) introducing an elution liquid in the cartridge by the aspiration-discharge means, for suspending the lysis means contained in the cartridge, and
g) mechanically lysing the microorganisms, by rotating the rotor, within the cartridge by the rotating means of said rotor, said rotor causing the lysis means to rotate, and
h) aspirating the elution liquid containing the nucleic acids of said microorganisms released during lysis, and
i) dispensing the elution liquid in a container for receiving and purification of nucleic acids.

16. The method as claimed in the preceding claim, comprising an additional stage for washing and purification of nucleic acids.

17. The method as claimed in claims 13 to 16, having an additional stage d') consisting of growing the microorganisms concentrated in the retention zone of the cartridge.

18. The method as claimed in the preceding claim, in which growing is achieved by incubation of the cartridge in a stove for a time in the range from 2 to 24 hours.

19. The method as claimed in one of claims 15 to 18, in which stages f) to i) are employed in the system for lysis of microorganisms and purification of nucleic acids as claimed in one of claims 9 to 12.

20. A method for lysis of microorganisms, said method having stages consisting of:
a) obtaining a cartridge in which microorganisms are concentrated in the retention zone of said cartridge,
b) placing the rotor in the cartridge, so as to obtain the device for lysis of microorganisms as claimed in one of claims 5 to 8,
c) introducing an elution liquid in the cartridge, for suspending the lysis means situated in the microorganism retention zone of the cartridge, and
d) mechanically lysing the microorganisms, by rotating the rotor within the cartridge, said rotor causing the means for lysis of microorganisms to rotate.

21. A method of extraction of nucleic acids from microorganisms, said method having stages consisting of:
a) obtaining a cartridge in which microorganisms are concentrated in the retention zone,
b) placing the rotor in the cartridge, so as to obtain the device for lysis of microorganisms as claimed in one of claims 5 to 8,
c) introducing an elution liquid in the cartridge, for suspending the lysis means situated in the microorganism retention zone of the cartridge, and
d) mechanically lysing the microorganisms, by rotating the rotor within the cartridge, said rotor causing the means for lysis of microorganisms to rotate, and
e) aspirating the elution liquid containing nucleic acids of said microorganisms released during lysis, and
f) dispensing the elution liquid in a container for receiving and purification of the nucleic acids.

22. The method as claimed in the preceding claim, comprising an additional stage for washing and purification of the nucleic acids.

23. A method for lysis of microorganisms contained in a liquid sample, said method having stages consisting of:
a) introducing the liquid sample containing said microorganisms into a cartridge, in the vicinity of the retention zone, so that said liquid sample leads to suspending of the lysis means situated in said microorganism retention zone of the cartridge,
b) placing the rotor in the cartridge, so as to obtain the device for lysis of microorganisms as claimed in one of claims 5 to 8,
c) mechanically lysing the microorganisms, by rotating the rotor within the cartridge, said rotor causing the lysis means on which the microorganisms are retained to be rotated.

24. A method of extracting the nucleic acids from microorganisms contained in a liquid sample, said method having stages consisting of:
a) introducing a liquid sample containing said microorganisms into a cartridge in the vicinity of the retention zone, so that said liquid sample leads to suspending of the lysis means situated in said microorganism retention zone of the cartridge,
b) placing the rotor in the cartridge, so as to obtain the device for lysis of microorganisms as claimed in one of claims 5 to 8,
c) mechanically lysing the microorganisms, by rotating the rotor within the cartridge, said rotor causing the lysis means to rotate,
d) aspirating the liquid sample containing the nucleic acids of said microorganisms released during lysis and
e) dispensing the liquid sample in a container for receiving and purification of the nucleic acids.

25. A method for extracting the nucleic acids from a sample of cellular tissue, said method having stages consisting of:
a) introducing the sample of cellular tissue into the cartridge in the presence of an elution liquid,
b) placing the rotor in the cartridge,
c) mechanically lysing the cells of the cellular tissue, by rotating the rotor within the cartridge, said rotor causing the lysis means contained in the cartridge to be rotated,
d) aspirating the elution liquid containing the nucleic acids from said cells, released during lysis and
e) dispensing the elution liquid in a container for receiving and purification of the nucleic acids.

26. The method as claimed in one of claims 24 or 25, comprising an additional stage for washing and purification of nucleic acids.

27. The use of the device for lysis of microorganisms as claimed in one of claims 5 to 8, for lysing cells of a sample of cellular tissue.

28. The use of the system for lysis of microorganisms and purification of the nucleic acids from said microorganisms as claimed in one of claims 9 to 12, for lysing cells from a sample of cellular tissue
